(19) Europäisches Patentamt
European Patent Office
Office européen des brevets

(11) Publication number: **0 076 089**
**A2**

(12) # EUROPEAN PATENT APPLICATION

(21) Application number: 82304980.4

(22) Date of filing: 22.09.82

(51) Int. Cl.³: **C 07 D 451/02**
C 07 D 451/14, C 07 D 498/08
C 07 D 513/08, A 61 K 31/435
A 61 K 31/46
//(C07D498/08, 265/00, 221/00),
(C07D513/08, 279/00, 221/00)

(30) Priority: **28.09.81 GB 8129282**
**21.11.81 GB 8135161**

(43) Date of publication of application:
**06.04.83 Bulletin 83/14**

(84) Designated Contracting States:
**BE CH DE FR GB IT LI NL SE**

(71) Applicant: **BEECHAM GROUP PLC**
**Beecham House Great West Road**
**Brentford Middlesex(GB)**

(72) Inventor: **Blaney, Frank Edward**
**19C Clissold Court**
**Green Way Close London N4 2EZ(GB)**

(72) Inventor: **Markwell, Roger Edward**
**22 Venmore Drive**
**Great Dunmow Essex(GB)**

(74) Representative: **Stott, Michael John et al,**
**European Patent Attorney Beecham Pharmaceuticals**
**Great Burgh Yew Tree Bottom Road**
**Epsom Surrey KT18 5XQ(GB)**

(54) **Azabicycloalkane derivatives, process for their preparation, and a pharmaceutical composition containing them.**

(57) Compounds of formula (I) or pharmaceutically acceptable salts thereof or solvates of the compounds or salts:

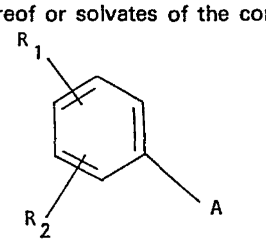

(I)

wherein:

$R_1$ is hydroxy, $C_{1-4}$ alkoxy or an in-vivo hydrolysable acyloxy group:

$R_2$ is hydrogen, $C_{1-4}$ alkyl, hydroxy, $C_{1-4}$ alkoxy or an in-vivo hydrolysable acyloxy group; or $R_1$ and $R_2$ together are $C_{1-2}$ alkylenedioxy;

A is a group

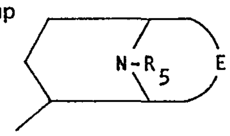

where E is

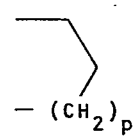

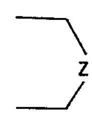

or

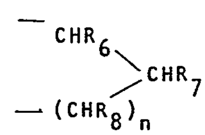

where

p is 0 to 2;

Z is O or S

n is 0 or 1; and

one of $R_6$ and $R_7$ when n=0 is $C_{1-4}$ alkyl and the other is hydrogen or $C_{1-4}$ alkyl or one of $R_6$, $R_7$ and $R_8$ when n=1 is $C_{1-4}$ alkyl and the other two are the same or different and are hydrogen or $C_{1-4}$ alkyl; and

$R_5$ is $C_{1-7}$ alkyl; a group $-(CH_2)_sR_{13}$ where s is 0 to 2 and $R_{13}$ is a $C_{3-8}$ cycloalkyl group; a group $-(CH_2)_tR_{14}$ where t is 1 or 2 and $R_{14}$ is $C_{2-5}$ alkenyl or a phenyl group optionally

./...

EP 0 076 089 A2

substituted by one or two substituents selected from $C_{1-4}$ alkoxy, trifluoromethyl, halogen, nitro, carboxy, esterified carboxy and $C_{1-4}$ alkyl optionally substituted by hydroxy, $C_{1-4}$ alkoxy, carboxy, esterified carboxy or *in vivo* hydrolysable acyloxy, or a thienyl group, having useful pharmacological activity, pharmaceutical compositions containing them, and a process for their preparation.

ACTIVE COMPOUNDS

This invention relates to novel compounds to pharmaceutical compositions containing them, and to a process for their preparation.

Racemic 3-(3-hydroxyphenyl)-N-n-propylpiperidine (3-PPP) is a known dopamine agonist which at lower dosages acts predominantly at presynaptic dopamine autoreceptors. At higher dosages one enantiomer acts as a postsynaptic dopamine agonist, whilst the other acts as a postynaptic dopamine antagonist. This compound is thus of potential use in the treatment of CNS disorders related to excess dopamine release.

We have now discovered a class of structurally distinct compounds which are similarly useful in the treatment of such disorders.

The present invention provides a compound of the formula (I), and pharmaceutically acceptable salts thereof:

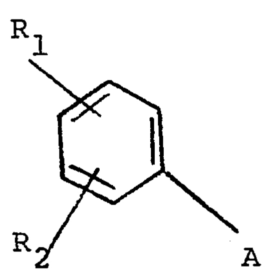

(I)

wherein:

$R_1$ is hydroxy, $C_{1-4}$ alkoxy or an in-vivo hydrolysable acyloxy group:

$R_2$ is hydrogen, $C_{1-4}$ alkyl, hydroxy, $C_{1-4}$ alkoxy or an in-vivo hydrolysable acyloxy group; or

$R_1$ and $R_2$ together are $C_{1-2}$ alkylenedioxy;

A is a group

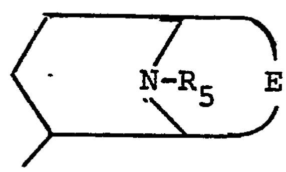

where E is

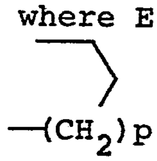

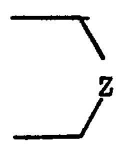

or

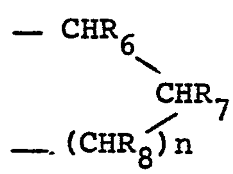

where

p is 0 to 2;

Z is O or S;

n is 0 or 1; and

one of $R_6$ and $R_7$ when n=0 is $C_{1-4}$ alkyl and the other is hydrogen or $C_{1-4}$ alkyl or one of $R_6$, $R_7$ and $R_8$ when n=1 is $C_{1-4}$ alkyl and the other two are the same or different and are hydrogen or $C_{1-4}$ alkyl; and

$R_5$ is $C_{1-7}$ alkyl; a group $-(CH_2)_sR_{13}$ where s is 0 to 2 and $R_{13}$ is a $C_{3-8}$ cycloalkyl group; a group $-(CH_2)_tR_{14}$ where t is 1 or 2 and $R_{14}$ is $C_{2-5}$ alkenyl or a phenyl group optionally substituted by one or two substituents selected from $C_{1-4}$ alkoxy, trifluoromethyl, halogen, nitro, carboxy, esterified carboxy and $C_{1-4}$ alkyl optionally substituted by hydroxy, $C_{1-4}$ alkoxy, carboxy, esterified carboxy or in vivo hydrolysable acyloxy:

A preferred value of E is $(CH_2)_2(CH_2)_p$.

When A is a group of formula (II) as defined, p is suitably 0 or 1, preferably 0.

When A is a group of formula (III) as defined Z is O or S, preferably O.

When A is a group of formula (IV) as defined, preferably each of $R_6$ and $R_7$ when n=0 and each of $R_6$, $R_7$ and $R_8$ when n=1 are in the exo-position.

Preferred examples for one of $R_6$ and $R_7$ when n=0 are methoxy, ethoxy, n-propoxy, methyl, ethyl, n-propyl, methoxycarbonyl and ethoxycarbonyl, hydroxy, hydroxymethyl or hydroxyethyl, methoxymethyl, methoxyethyl, ethoxymethyl, ethoxyethyl, acetylmethyl and acetylethyl. The most preferred is methyl.

Preferred examples for one of $R_6$, $R_7$ and $R_8$ when n=1 are methyl, ethyl and n-propyl.

Preferred examples for the other of $R_6$ and $R_7$ when n=0 and for the other two of $R_6$, $R_7$ and $R_8$ when n=1 are hydrogen, methyl, ethyl and n- and iso-propyl.

Examples of $R_1$ and $R_2$ include hydroxyl, methoxy, ethoxy, n- and iso-propoxy and in-vivo hydrolysable acyloxy groups, such as $C_{1-6}$ alkanoyloxy, for example acetoxy, propionoxy, and n- and iso- butyroxy, 2,2-dimethylpropanoyloxy, benzoyloxy optionally substituted in the phenyl ring by one or two substituents selected from $C_{1-4}$ alkyl, $C_{1-4}$ alkoxy, trifluoromethyl, halogen or nitro, labile sulphonate groups for example $C_{1-4}$ alkanesulphonyloxy, e.g. methanesulphonyloxy, or benzenesulphonyloxy optionally substituted as for benzoyloxy above, e.g. toluenesulphonyloxy; and together, ethylenedioxy.

$R_2$ may also be hydrogen or $C_{1-4}$ alkyl. Suitable $C_{1-4}$ alkyl groups include methyl, ethyl and n- and iso-propyl, preferably methyl.

$R_1$ and/or $R_2$ will often be hydroxyl, methoxy, 2,2-dimethylpropanoyloxy or benzoyloxy.

$R_2$ will often be hydrogen or the same as $R_1$.

$R_1$ will usually be in the 3-position and $R_2$ will usually be in the 4- or 5-positions (standard numbering for the phenyl ring system).

Suitable examples of $R_5$ when $C_{1-7}$ alkyl include methyl, ethyl, n- and iso-propyl and n-, sec-, iso-, and tert-butyl, n-pentyl, n-hexyl, n-heptyl and 3-methylbutyl.

Within $C_{1-7}$ radicals, $C_{1-3}$ alkyl are useful.

Suitable examples of $R_5$ when $C_{1-3}$ alkyl include methyl, ethyl, n- and iso-propyl, particularly n-propyl and iso-propyl.

Suitable examples of $R_5$ when $C_{4-7}$ alkyl include n-pentyl, n-hexyl and n-heptyl, especially those of the form $(CH_2)_uR_{15}$ wherein u is 1 or 2 and $R_{15}$ is a secondary or tertiary $C_{3-6}$ alkyl group such as n-sec- and tert-butyl, n-pentyl, n-hexyl and n-heptyl, and especially iso-butyl, 3-methylbutyl, 2,2-dimethylpropyl and 3,3-dimethylbutyl, 4-methylpentyl and 5-methylhexyl.

When $R_5$ is a group $-(CH_2)_sR_{13}$ as defined, suitable examples of $R_{13}$ include $C_{5-8}$ cycloalkyl, preferably cyclohexyl, cyclopentyl or cyclopropyl.

When $R_5$ is a group $-(CH_2)_tR_{14}$ and defined, t is 1 or 2.

In such a group $R_5$ when $R_{14}$ is $C_{2-5}$ alkenyl, suitable examples thereof include vinyl, prop-1-enyl, prop-2-enyl, 1-methylvinyl, but -1-enyl, but-2-enyl, but-3-enyl, 1-methylenepropyl, 1-methylprop-1-enyl, and 1-methylprop-2-enyl, in E and Z forms. Such $R_5$ is favourably allyl.

When $R_{14}$ is optionally substituted phenyl as defined above, suitable examples of such optional phenyl substituents include methyl, ethyl, n- and iso-propyl, n, sec- and tert-butyl, preferably methyl, optionally substituted by hydroxyl, methoxy, ethoxy, n- and iso-propoxy and in-vivo hydrolysable acyloxy groups, such as $C_{1-6}$ alkanoyloxy, for example acetoxy, propionoxy, and n- and iso-butyroxy, 2,2-dimethylpropanoyloxy, benzoyloxy optionally substituted in the phenyl ring by one or two substituents selected from $C_{1-4}$ alkyl, $C_{1-4}$ alkoxy, trifluoromethyl, halogen or nitro, and labile sulphonyloxy groups for example $C_{1-4}$ alkanesulphonyloxy, e.g. methanesulphonyloxy, or benzenesulphonyloxy optionally substituted as for benzoyloxy, often hydroxyl, methoxy, 2,2-dimethyl-propanoyloxy or

benzoyloxy; methoxy, ethoxy, n- and iso-propoxy; $CF_3$, fluoro, chloro or bromo. Preferably $R_{14}$ when optionally substituted phenyl is unsubstituted. When $R_{14}$ is thienyl it may be 2- or 3-thienyl.

Values for $R_5$ include optionally substituted benzyl and phenethyl as hereinbefore defined and thienylmethyl (also called thenyl). Optionally substituted benzyl and phenethyl are favoured, preferably benzyl and phenethyl.

The compounds of the formula (I) may form acid addition salts at the bicyclic moiety nitrogen atom.

The pharmaceutically acceptable salts of the compounds of the formula (I) include acid addition salts with convention acids such as hydrochloric, hydrobromic, phosphoric, sulphuric, citric, tartaric, lactic and acetic acid and the like.

The compounds of the formula (I) and their salts can also form solvates and the invention extends to such solvates.

It will of course be realised that the compounds of the formula (I) have at least one chiral centre, and thus are capable of existing in a number of stereoisomeric forms. In particular it will be seen that the phenyl-bicycle linkage has an exo or endo orientation with respect to the bicycle.

The invention extends to each of these stereoisomeric forms including enantiomers and to mixtures thereof (including racemates).

A group of compounds within those of formula (I) is those wherein E is

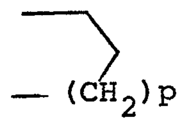

Another group of compounds within those of formula (I) is of formula (II).

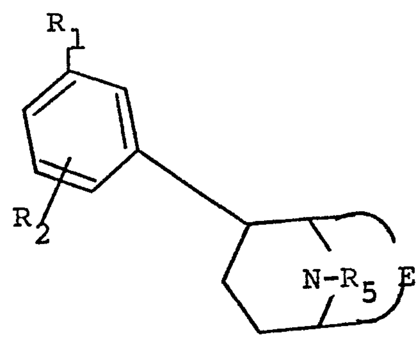

(II)

wherein:

the variables are as defined in formula (I).

Suitable and preferred $R_1$, $R_2$ and $R_5$ are as so described under formula (I).

A group of compounds within those of formula (II) is of formula (III):

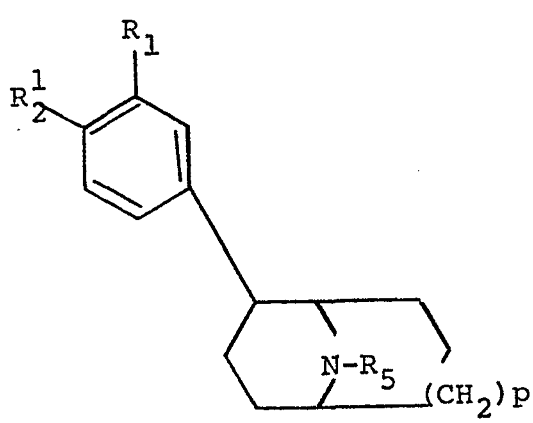

(III)

wherein:

$R_2^1$ is hydroxy, $C_{1-4}$ alkoxy or in-vivo hydrolysable acyloxy; and the remaining variables are as defined in formula (I).

Suitable and preferred $R_1$ and $R_2^1$ are as so described for $R_1$ and relevant $R_2$ under formula (I).

Suitable and preferred remaining variables are as so described under formula (I).

A sub-group of compounds within formula (III) is of formula (IV):

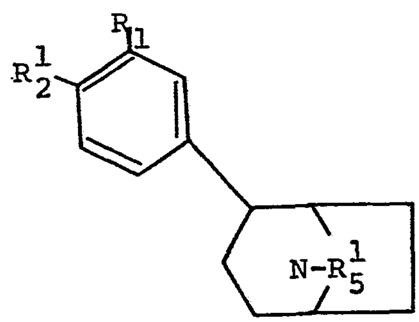

(IV)

wherein $R_5^1$ is $C_{1-3}$ alkyl or allyl; and

$R_1$ and $R_2^1$ are as defined in formula (III)

Suitable examples of $R_5^1$ are allyl and as so described for $R_5$ $C_{1-3}$ alkyl under formula (I); n-propyl and iso-propyl are preferred.

Suitable examples of $R_1$ and $R_2^1$ are as so described under formula (III).

Another sub-group of compounds within those of formula (III) are those of the formula (V):

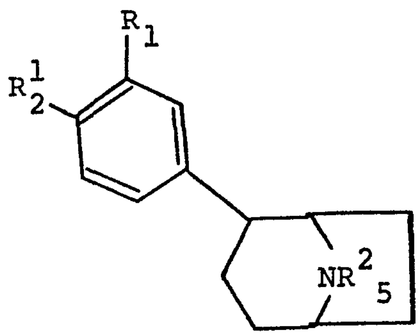

(V)

wherein $R^2_5$ is a group $-(CH_2)_tR^1_{14}$ where t is 1 or 2 and $R_{14}$ is optionally substituted phenyl as defined in formula (I); or thienylmethyl; and $R_1$ and $R^1_2$ are as defined in formula (III).

Suitable and preferred $R^2_5$ are as so described for the corresponding $R_5$ groups under formula (I).

$R^2_5$ benzyl andphenethyl are preferred.

Suitable and preferred $R_1$ and $R^1_2$ are as so described under formula (III).

Another sub-group of compounds within those of formula (III) are those of the formula (VI):

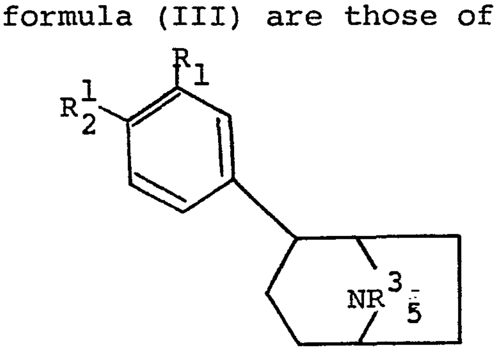

(VI)

wherein

$R^3_5$ is $C_{4-7}$ alkyl or $-(CH_2)_sR_{13}$ where s is 0 to 2 and $R_{13}$ is $C_{3-8}$ cycloalkyl; and $R_1$ and $R^1_2$ are as defined in formula (III).

Suitable and preferred $R_5^3$ are as so described for the corresponding $R_5$ groups under formula (I).

Suitable and preferred $R_1$ and $R_2^1$ are as so described under formula (III).

A group of compounds within those of formula (VI) is that wherein $R_5^3$ is $(CH_2)_sR_{13}$ as defined or $(CH_2)_uR_{15}$ where s, u, $R_{13}$ and $R_{15}$ are as defined.

Examples of $R_5^3$ in these compounds include iso-butyl, 3-methylbutyl, 2,2-dimethylpropyl, 3,3-dimethylbutyl, cyclopropylethyl, and cyclopropylmethyl.

A further sub-group of compounds within formula (III) is of formula (VII):

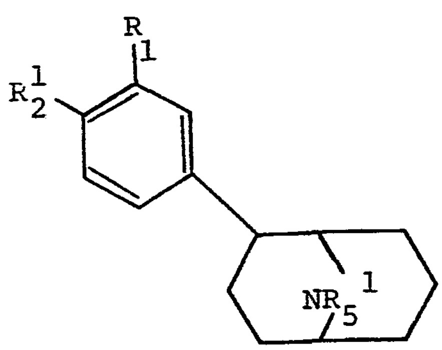

(VII)

wherein the variables are as defined in formula (IV).

Suitable examples of $R_5^1$ are as so described under formula (IV).

Suitable examples of $R_1$ and $R_2^1$ are as so described under formula (IV).

Another sub-group of compounds within those of the formula (III) of interest are those of the formula (VIII):

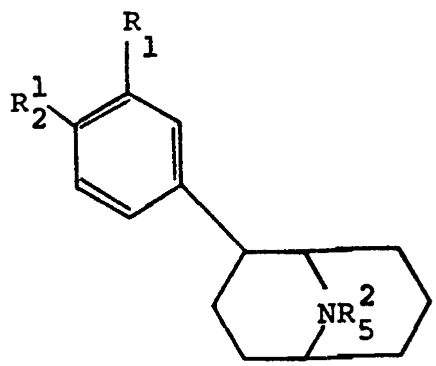

(VIII)

wherein the variables are as defined in formula (V)]

Suitable and preferred examples of $R_5^2$ are as so described under formula (V).

Suitable and preferred examples of $R_1$ and $R_2^1$ are as so described under formula (III).

Another sub-group of compounds within those of the formula (III) of interest are those of the formula (IX).

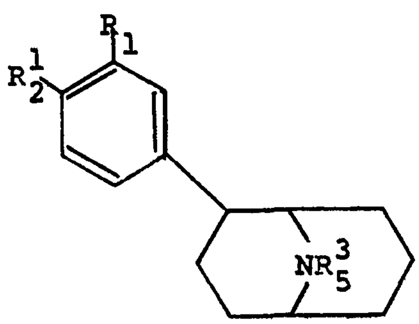

(IX)

wherein the variables are as defined in formula (VI).

Suitable and preferred examples of $R_5^3$ are as so described under formula (VI).

Suitable and preferred examples of $R_1$ and $R_2^1$ are as so described under formula (III).

A second group of compounds within formula (II) is of formula (X):

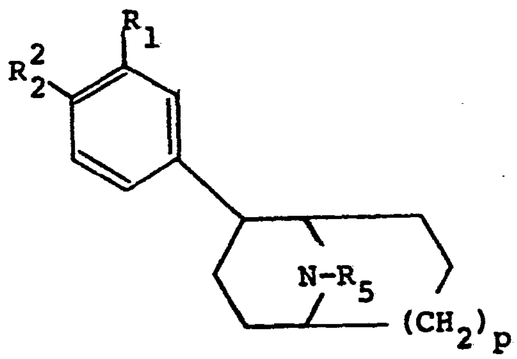

(X)

wherein

$R_2^2$ is hydrogen or $C_{1-4}$ alkyl; and the remaining variables are as defined in formula (I).

Suitable and preferred $R_1$ and $R_2^2$ are as so described for $R_1$ and relevant $R_2$ under formula (I).

Suitable and preferred remaining variables are as so described under formula (I).

A sub-group of compounds within formula (X) is of formula (XI):

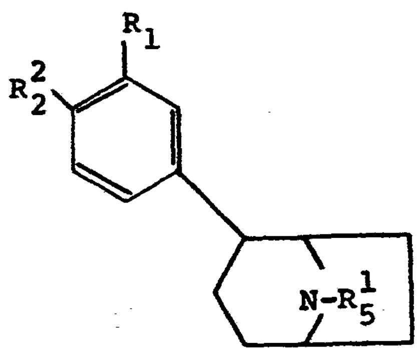

(XI)

wherein

$R_5^1$ is $C_{1-3}$ alkyl or allyl; and

$R_1$ and $R_2^2$ are as defined in formula (X).

Suitable examples of $R_5^1$ are allyl and as so described for $R_5$ $C_{1-3}$ alkyl under formula (I); n-propyl and iso-propyl are preferred.

Suitable examples of $R_1$ and $R_2^2$ are as so described under formula (X).

Another sub-group of compounds within those of formula (X) are those of the formula (XII):

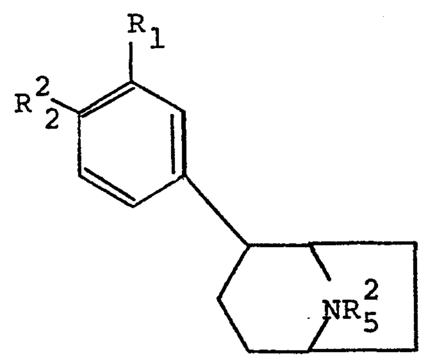

(XII)

wherein

$R_5^2$ is a group $-(CH_2)_tR_{14}^1$ wherein t is 1 or 2 and $R_{14}^1$ is optionally substituted phenyl as defined in formula (I); or thienylmethyl; and $R_1$ and $R_2^2$ are as defined in formula (X).

Suitable and preferred $R_5^2$ are as so described for the corresponding $R_5$ groups under formula (I).

$R_5^2$ benzyl and phenethyl are preferred.

Suitable and preferred $R_1$ and $R_2^2$ are as so described under formula (X).

Another sub-group of compounds within those of formula (X) are those of the formula (XIII):

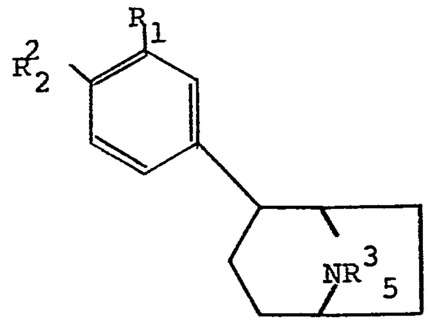

(XIII)

$R_5^3$ is $C_{4-7}$ alkyl or $-(CH_2)_sR_{13}$ where s is 0 to 2 and $R_{13}$ is $C_{3-8}$ cycloalkyl; and $R_1$ and $R_2^2$ are as defined in formula (X).

Suitable and preferred $R_5^3$ are as so described for the corresponding $R_5$ groups under formula (I).

Suitable and preferred $R_1$ and $R_2^2$ are as so described under formula (VIII).

A group of compounds within those of formula (XIII) is that wherein $R_5^3$ is $(CH_2)_sR_{13}$ as defined or $(CH_2)_uR_{15}$ where s, u, $R_{13}$ and $R_{15}$ are as defined.

Examples of $R_5^3$ in these compounds include iso-butyl, 3-methylbutyl, 2,2-dimethylpropyl, 3,3-dimethylbutyl cyclopropylethyl, and cyclopropylmethyl.

A sub-group of compounds within those of formula (X) of interest are of formula (XIV):

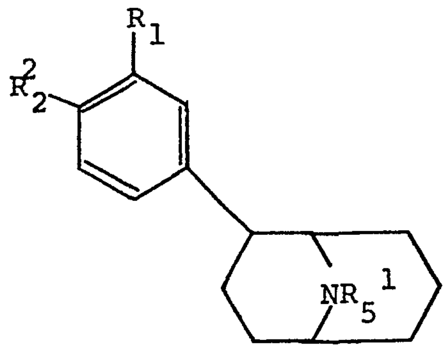

(XIV)

wherein the variables are as defined in formula (III).

Suitable examples of $R_5^1$ are as so described under formula (IV).

Suitable examples of $R_1$ and $R_2^2$ are as so described under formula (X).

Another sub-group of compounds within those of the formula (X) of interest are those of the formula (XV):

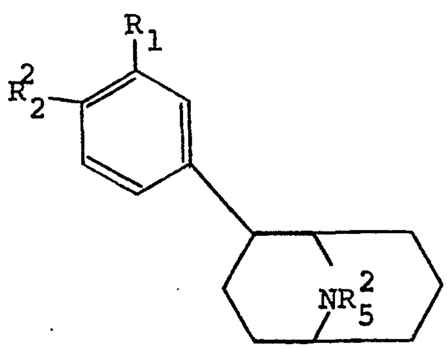

(XV)

wherein the variables are as defined in formula (V).

Suitable and preferred examples of $R_5^2$ are as so described under formula (V).

Suitable and preferred examples of $R_1$ and $R_2^2$ are as so described under formula (X).

A further sub-group of compounds within those of the formula (X) of interest are those of the formula (XVI):

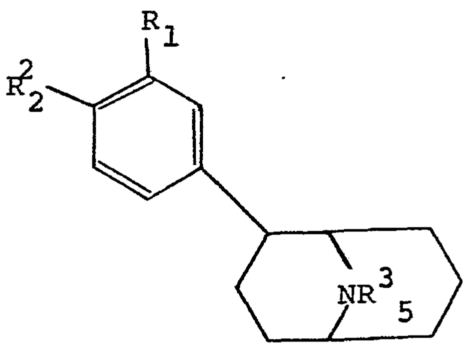

(XVI)

wherein the variables are as defined in formula (V1)

Suitable and preferred examples of $R_5^3$ are as so described under formula (V1).

Suitable and preferred examples of $R_1$ and $R_2^2$ are as so described under formula (X).

A third group of compounds within those of formula (II) are those of the formula (XVII):

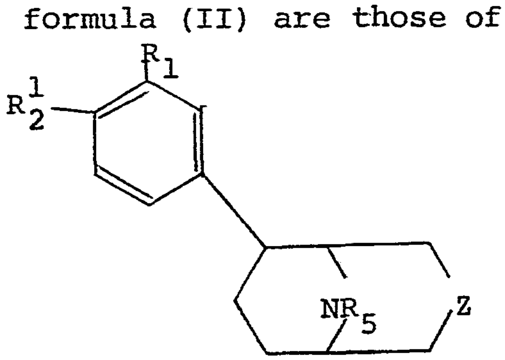

(XVII)

wherein the variables are as defined in formulae (1) and (III)

Suitable and preferred examples of $R_5$ are as so described under formula (I).

Suitable and preferred examples of $R_1$ and $R_2^1$ are as so described under formula (III).

Z is preferably O.

A sub-group of compounds within those of formula (XVII) are those of the formula (XVII).

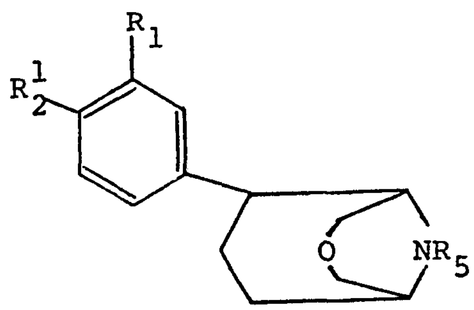

(XVIII)

wherein the variables are as defined in formula (XVII).

Suitable and preferred variables are as so described under formula (XVII).

Suitable and preferred $R_1$ and $R_2^1$ are as so described under formula (III).

Another sub-group of compounds within those of formula (XVII) are those of the formula (XIX):

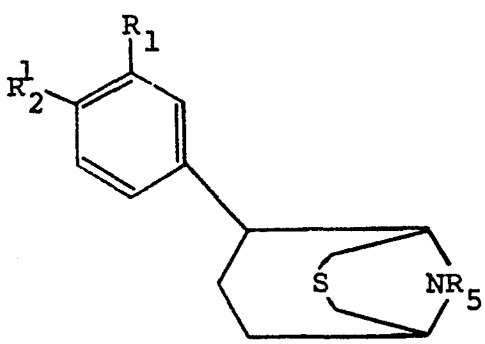

(XIX)

wherein the variables are as defined in formula (XVII).

Suitable and preferred variables are as so described under formula (XVII).

Suitable and preferred $R_1$ and $R_2^1$ are as so described under formula (III).

A fourth group of compounds within formula (II) is of formula (XX):

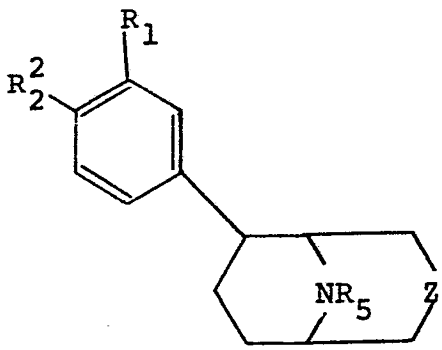

(XX)

wherein

$R_2^2$ is hydrogen or $C_{1-4}$ alkyl; and the remaining variables are as defined in formula (I).

Suitable and preferred $R_1$ and $R_2^2$ are as so described under formula (X).

Suitable and preferred remaining $R_5$ are as so described under formula (I).

Z is preferably O.

A sub-group of compounds within formula (XX) is of formula (XXI):

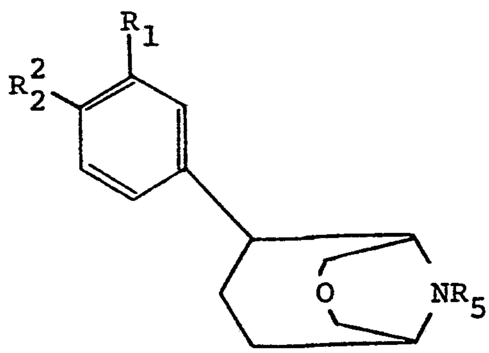

(XXI)

wherein the variables are as defined in formula (XX).

Suitable examples of $R_1$, $R_2^2$ and $R_5$ are as so described under formula (XX).

Another sub-group of compounds within those of formula (XX) are those of the formula (XXII).

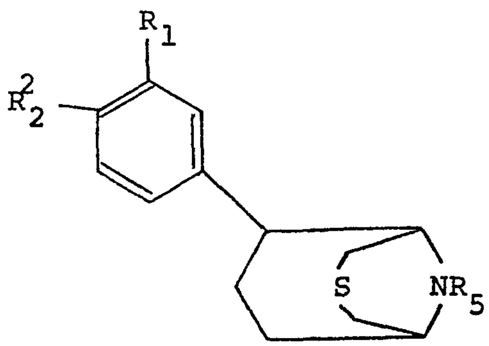

(XXII)

wherein the variables are as defined in formula (XX).

Suitable and preferred $R_1$, $R_2^2$ and $R_5$ are as so described under formula (XX).

A fifth group of compounds within those of formula (II) is of formula (XXIII):

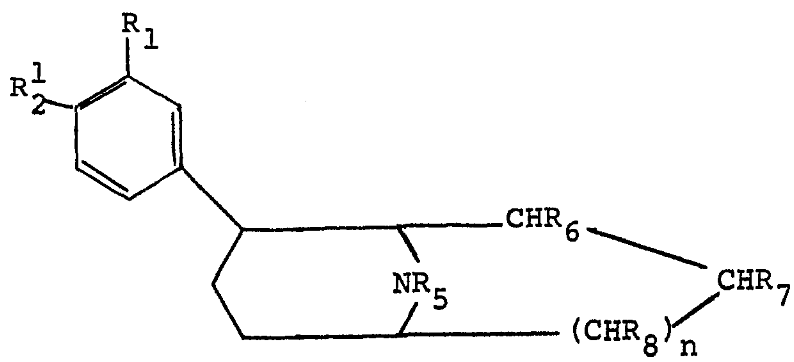

(XXIII)

wherein

$R_2^1$ is hydroxy, $C_{1-4}$ alkoxy or in-vivo hydrolysable acyloxy; and the remaining variables are as defined in formula (I)

Suitable and preferred $R_1$ and $R_2^1$ are as so described under formula (III).

Suitable and preferred remaining variables are as so described under formula (I).

A sub-group of compounds within formula (XXIII) is of formula (XXIV):

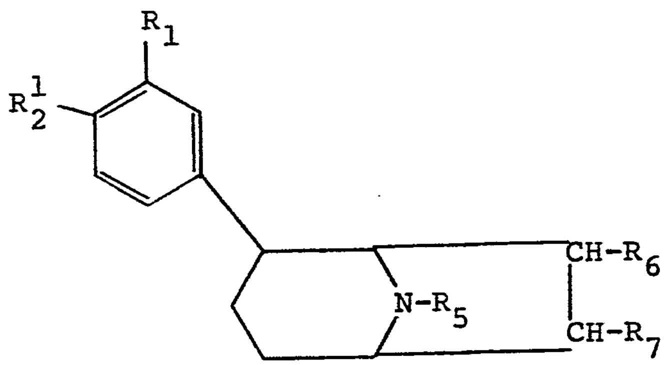

(XXIV)

wherein the variables are as defined in formula (XXIII).

One of $R_6$ and $R_7$ is often methyl or ethyl, and the other is hydrogen, methyl or ethyl.

Preferred $R_5$ and suitable examples of $R_1$ and $R_2^1$ are as so described under formula (III).

Another sub-group of compounds within those of formula (XXIII) are those of the formula (XXV):

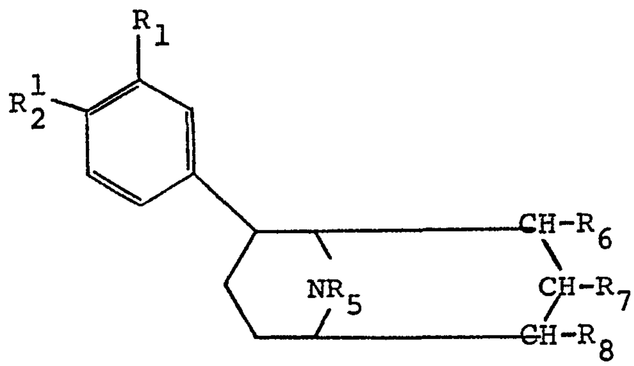

(XXV)

wherein the variables are as defined in formula (XXIII).

One of $R_6$, $R_7$ and $R_8$ is often methyl or ethyl and the other two are the same or different and are methyl, ethyl or hydrogen.

Preferred $R_5$ are as so described under formula (I).

Suitable and preferred remaining variables are as so described under formula (III).

Within formula (II) is a sixth group of compounds of formula (XXVI):

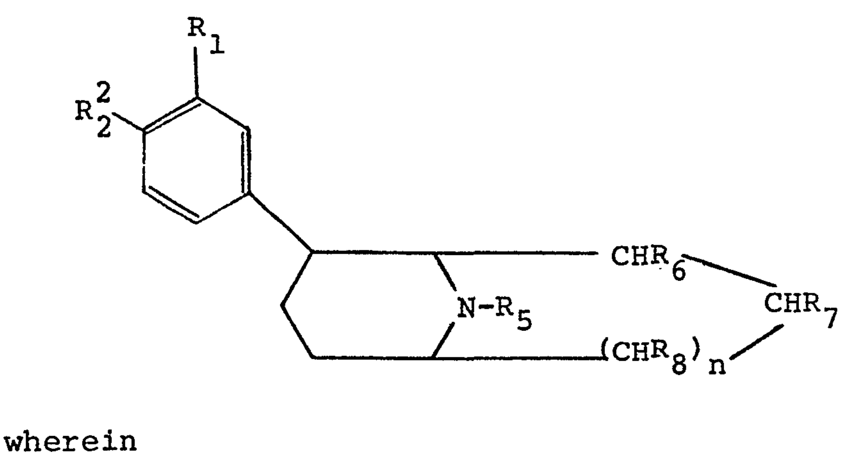

(XXVI)

wherein

$R_2^2$ is hydrogen or $C_{1-4}$ alkyl; and the remaining variables are as defined in formula (I).

Suitable and preferred $R_1$ and $R_2^2$ are as so described under formula (X).

Suitable and preferred remaining variables are as so described under formula (I).

Within formula (XXVI) is a sub-group of compounds of formula (XXVIIA)

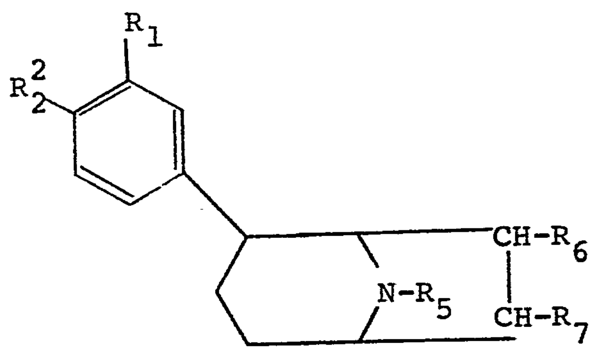

(XXVIIA)

One of $R_6$ and $R_7$ is often methyl or ethyl and the other is hydrogen, methyl or ethyl.

Preferred $R_5$ are as so described under formula (I).

Suitable examples of $R_1$ and $R_2^2$ are as so described under formula (X).

Another sub-group of compounds within those of formula (XXVI) are those of the formula (XXVIIB):

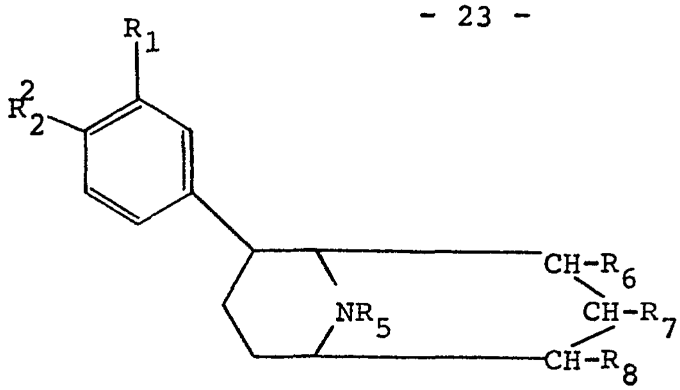

(XXVIIB)

wherein the variables are as defined in formula (XXVI).

One of $R_6$, $R_7$ and $R_8$ is often methyl or ethyl and the other two are the same or different and are methyl, ethyl or hydrogen.

Preferred $R_5$ are as so described under formula (I).

Suitable and preferred $R_1$ and $R_2^2$ are as so described under formula (X).

The invention also provides a process for the preparation of a compound of the formula (I), which process comprises the reduction of a compound of the formula (XXVIII):

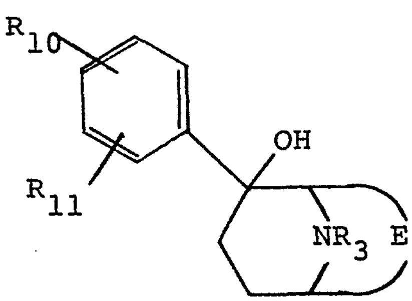

(XXVIII)

wherein E is

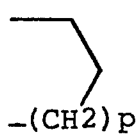

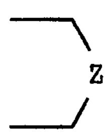

or

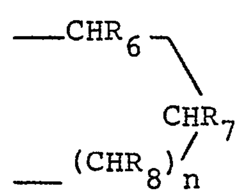

wherein the variables are as hereinbefore defined.

$R_{10}$ is hydroxyl or protected hydroxyl;

$R_{11}$ is hydrogen, $C_{1-4}$ alkyl, hydroxyl or protected hydroxyl;

$R_3$ is $R_5$ as defined in formula (I) or $C_{1-7}$ alkanoyl; a group $-CO(CH_2)_xR_{13}$ where x is 0 or 1 and $R_{13}$ is as defined in formula (I); or a group $-CO(CH_2)_yR_{14}$ where y is 0 or 1 and $R_{14}$ is as defined in formula (I) excluding $C_{2-5}$ alkenyl

and the remaining variables are as defined in formula (I):

and as necessary converting $R_3$ to $R_5$;

as desired or necessary converting $R_{10}$ or $R_{11}$ to $R_1$ or $R_2$ respectively; optionally converting $R_5$ to other $R_5$; and optionally salifying the resulting compound of the formula (I).

An $R_{10}$ or $R_{11}$ protected hydroxyl group is a conventional group readily convertible after a desired reaction to a hydroxyl group, and examples include $C_{1-4}$ alkoxy and in-vivo hydrolysable acyloxy as defined and described for $R_1$ and $R_2$ in and under formula (I); and benzyloxy optionally substituted in the phenyl ring as for $R_5$ when benzyl or phenethyl.

Reduction may conveniently be effected in a single step using a metal-electron acceptor reductive couple, such as lithium in liquid ammonia.

When lithium-liquid ammonia is used the reaction is conveniently quenched on completion using ammonium chloride.

Alternatively, the reduction may be effected in two steps without necessarily isolating the relevant intermediate.

Compounds of the formula (XVIII) may be converted by dehydration to compounds of formula (XXIX):

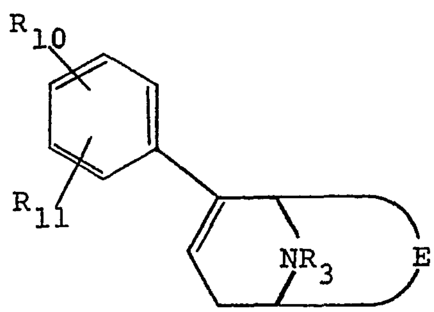

(XXIX)

wherein the variables are as defined in formula (XVIII):

Dehydration may be effected using a dilute strong acid, for example a dilute mineral acid such as dilute sulphuric acid. Moderate heating to a non-extreme elevated temperatures for example between ambient and 90°C, is advantageous. Extremes of acidity and temperature may cause polymerisation of the product and should therefore be avoided. Other suitable acids include strong organic acids in organic solvents, such as tosic acid in toluene.

The compound of the formula (XXIX) is then reduced and as necessary $R_3$ converted to $R_5$, as desired or necessary, $R_{10}$ or $R_{11}$ converted to $R_1$ or $R_2$ respectively or $R_5$ to other $R_5$ and the resulting compound of the formula (I) optionally salified.

This process step forms an aspect of the present invention.

The reduction of the compound of the formula (XXIX) is conveniently effected with conventional transition - metal catalysed hydrogenation using mild reaction conditions when $R_3$ is optionally substituted benzyl as defined or $R_{10}$ or $R_{11}$ is optionally substituted benzyloxy as defined, and if it is desired to avoid removal of $R_3$, $R_{10}$ or $R_{11}$. Platinum oxide or rhodium catalyst at atmospheric pressure or a slight excess thereover is suitable. For other $R_3$, $R_{10}$ or $R_{11}$, or if $R_3$, $R_{10}$ or $R_{11}$ retention is not desired, less mild conditions may be used, for example palladium - or platinum - charcoal, at atmospheric pressure or a slight excess thereover. Ambient temperatures are apt, as are dry inert polar solvents, such as dry ethanol. If $R_3$ optionally substituted benzyl is removed the resultant secondary amine NH may be converted to $NR_5$ by conventional alkylation or by acylation and reduction.

When $R_3$ is $C_{1-7}$ alkanoyl, $C_{3-8}$ cycloalkyl $C_{1-2}$ alkanoyl or $C_{3-7}$ alkenoyl, conversion to the corresponding $R_5$ may be effected by reductive acylation, for example by reaction with the corresponding acid in the presence of an inorganic hydride reductant such as sodium borohydride.

Conversion to $R_5$ methyl may be effected with formaldehyde in the presence of a mild reductant such as sodium cyanoborohydride in an inert highly polar solvent such as acetonitrile.

When $R_3$ is aryl $C_{1-2}$ alkanoyl as defined it is more convenient to effect acylation with the acyl halide and to reduce the product with a strong inorganic hydride reductant such as lithium aluminium hydride.

When $R_{10}$ or $R_{11}$ when a protected hydroxyl group, is of the form $R_{12}O$, where $R_{12}$ is $C_{1-4}$ alkyl, conversion of $R_{10}$ or $R_{11}$ to $R_1$ or $R_2$ hydroxyl respectively is conveniently carried out by treatment with pyridine hydrochloride, boron tribromide or boron triiodide or iodotrimethylsilane, or by warming with aqueous hydrobromic acid.

When $R_{12}$ is $C_{1-6}$ alkanoyl or benzoyl optionally substituted as defined deprotection may be effected conventionally, for example by acidic or basic hydrolysis.

When $R_{12}$ is optionally substituted benzyl as defined above conversion is conveniently effected by conventional methods such as transition metal catalysed hydrogenolysis using for example palladium - or platinum - charcoal, at atmospheric pressure or a slight excess thereover. This method is not apt for $R_5$ optionally substituted benzyl, which will also tend to be removed.

Compounds of the formula (I) wherein $R_1$ and optionally $R_2$ are $C_{1-4}$ alkoxy or in-vivo hydrolysable acyloxy are derivable from those wherein $R_1$ and $R_2$ are hydroxy by conventional O-alkylation or esterification reactions respectively.

The reaction may be carried out under conventional O-alkylation conditions, using a compound of the formula $R_{12}{}^1Q$ where $R^1{}_{12}$ is $C_{1-4}$ alkyl and Q is a group readily displaced by a nucleophile. Suitable examples of Q include halide such as Cl, Br or I or labile sulphonate groups such s $OSO_2CH_3$ or $OSO_2.C_6H_4.p\ CH_3$.

The reaction is generally effected in an inert solvent, at a non-extreme temperature such as ambient or slightly elevated temperature, for example solvent reflux temperature.

It will be appreciated by the skilled man that O-alkylation of compounds of the formula (I) may also lead to quaternary N-alkylation of the $=NR_5$ moiety unless the N-atom is protected, for example by carrying out acylation in the presence of a strong acid such as trifluoroacetic acid, so that the nitrogen function is protected by protonation. The acid may conveniently also act as the solvent.

For in-vivo hydrolysable esters the reaction may be carried out under conventional esterification condition using a compound of the formula $R^2_{12}W$ wherein $R^2_{12}$ is an acyl group capable of forming an in-vivo hydrolysable acyloxy group and W is for example halide, such as chloride; acyloxy, such as $C_{1-4}$ alkanoyloxy; or hydroxyl.

$R^2_{12}$ may typically be the acyl group corresponding to $R_1$ and $R_2$ in-vivo hydrolysable acyloxy groups described under formula (I).

When W is halide the reaction is generally carried out in the presence of a base; when W is hydroxyl it is generally effected in the presence of a dehydrating agent such as dicyclohexylcarbodiimide.

The reaction is generally effected in an inert solvent at non-extreme temperatures such as ambient or slightly elevated temperature, for example solvent reflux temperature.

It will be appreciated that all the above interconversions may also be carried out in intermediates which are not of formula (I) or in the compounds of formulae (XXVIII) or (XXIX) themselves.

From the foregoing it will be appreciated that this invention also provides a third process for the preparation of a compound of the formula (I) or a pharmaceutically acceptable salt thereof, which process comprises the deprotection of a compound of the formula (XXX).

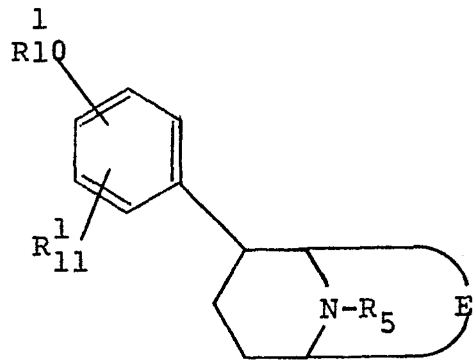

(XXX)

wherein:

$R^1_{10}$ is protected hydroxy;

$R^1_{11}$ is hydrogen, $C_{1-4}$ alkyl or protected hydroxy; and the remaining variables are as defined in formula (I); and thereafter optionally O-alkylating or O-acylating with an acyl group capable of forming an in-vivo hydrolysable acyloxy group, the resultant compound; and optionally salifying the resultant compound.

Suitable process conditions are as so described for the relevant first-process steps hereinbefore.

The reduction of the compound of formula (XXIX) may remove benzyl groups as defined.

The present invention thus provides a fourth process for the preparation of a compound of the formula (I), comprising reacting a compound of formula (XXXI).

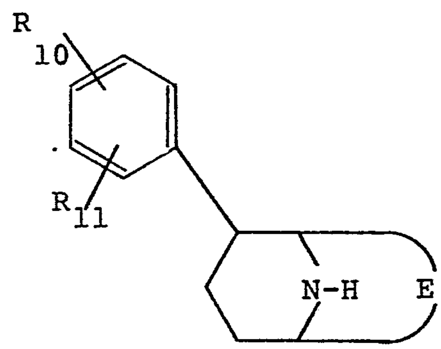

(XXXI)

with a compound of the formula (XXXII):

$$Q - R_3 \qquad (XXXII)$$

wherein:

$R_{10}$ is hydroxy or protected hydroxy;

$R_{11}$ is hydrogen, $C_{1-4}$ alkyl, hydroxy or protected hydroxy;

Q is a group readily displaceable by a nucleophile; and the remaining variables are as hereinbefore defined;

and as necessary converting $R_3$ to $R_5$.

as desired or necessary converting $R_{10}$ or $R_{11}$ to $R_1$ or $R_2$ respectively; converting $R_1$, $R_2$ or $R_5$ to other $R_1$, $R_2$ or $R_5$; and optionally salifying the resulting compound of the formula (I).

Suitable examples of Q include halide such as Cl, Br or I, when $R_3$ is $R_5$ as defined Q may also be labile sulphonyloxy such as $OSO_2CH_3$ or $OSO_2.C_6H_4.p.CH_3$, when $R_3$ is other than $R_5$ Q may also be $C_{1-6}$ alkanoyloxy such as acetoxy, or hydroxy.

The condensation of the compounds of the formulae (XXXI) and (XXXII) is conveniently effected in an inert aprotic solvent such as an ether, for example THF, or a halohydrocarbon, for example dichloromethane, or in a mixture of such solvents, at a non-extreme temperature.

Compounds of formula (XXXI) are novel, and as such form an aspect of the invention.

The reaction generally eliminates an acid, and it is thus often preferable to effect it in the presence of a base such as a mild inorganic base, for example potassium carbonate or a stronger organic base, for example triethylamine.

The variable interconversions may be effected as hereinbefore described.

The acid addition salts of compounds of the formula (I) may be prepared in entirely conventional manner by reacting a compound of the formula (I) in base form with the chosen acid.

The compound of formula (XXVIII) is conveniently prepared as described hereinafter and reacted in situ without intermediate isolation.

Compounds of the formula (XXVIII) may be prepared by the reaction of a compound of formula (XXXIII):

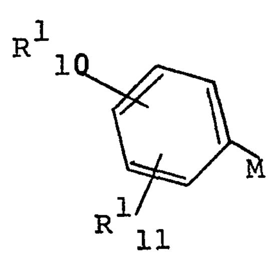

(XXXIII)

wherein:

$R_{10}^{1}$ is protected hydroxyl;

$R_{11}^{1}$ is hydrogen, $C_{1-4}$ alkyl or protected hydroxyl;

and

M is a magnesium (II) halide group, preferably magnesium (II) bromide, or lithium with a compound of the formula (XXXIV):

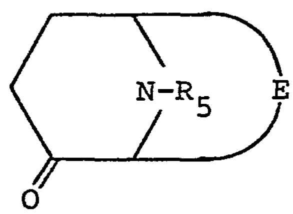

(XXXIV)

or

wherein the variables are as defined in formula (I), and thereafter as defined or necessary converting $R_{10}^{1}$ or $R_{11}^{1}$ to $R_{10}$ or $R_{11}$ as defined.

When M is a magnesium (II) halide group, the compound of formula (XXVIII) may be prepared in situ under conventional conditions for Grignard reagents. Those are: reaction of the halide, preferably the bromide, corresponding to the compound of formula (XXXIII) with a molar equivalent or excess of dry, grease-free magnesium particles in a dry ether, for example THF dimethoxyethane or diethyl ether, free of protic solvents. THF is a preferred solvent. The presence of trace quantities of dibromoethane may be advantageous. Ambient and non-extreme depressed temperatures are suitable, for example between ambient and -15$^{o}$C, although gentle initiative heating may be advantageous.

When M is lithium, the compound of formula (XXVIII) may be prepared in situ under conventional indirect metallation conditions, for example by reaction of the above corresponding

halide, preferably the bromide with n-butyl lithium. Temperatures of ambient to $-60^{o}C$ are suitable. The completed reaction is conveniently quenched with water.

It will be appreciated that any hydroxy group in $R_6$ is best protected and if desired deprotected after the coupling, as described hereinbefore.

Compounds of the formula (XXXIII) are derivable by conventional metallation or Grignard reactions from the corresponding compound where M is replaced by halide, preferably bromide.

These corresponding halo compounds are known compounds or derivable conventionally from or preparable analogously to known compounds.

Depending on reaction steric factors the compounds of the formulae (I) or (XXVIII), (XXX) and (XXXI) may be synthesised having an axial or equatorial substitution orientation at the azabicycle 2-position, or as a mixture of such forms in infinitely variable ratio. By way of example the reduction of a compound of the formula may give a mixture of both isomers. Such mixtures may be separated conventionally, e.g. by chromatography or fractional crystallisation. The latter method on an acid addition salt is especially useful where the corresponding compound of formula (XXVIII) is liquid under ambient conditions.

When reaction conditions cause the production of the axial isomer, this may if desired be converted to the corresponding equatorial isomer by treatment with a strong base, for example butyl lithium, optionally complexed with tetramethylethylenediamine.

Each such diastereomer also exists in two enantiomeric forms by virtue of the non-symmetric substitution of the azabicycle.

Specific enantiomers may be obtained by using chiral starting materials, or compounds of the formula (I) may be synthesised from racemic intermediates and their enantiomers resolved conventionally e.g. by salification with a chiral acid and salt separation.

It is often convenient to synthesise the compounds of the formula (I) non-stereospecifically and non-chirally from diastereomeric and/or enantiomeric mixtures and to separate and/or resolve the compounds of the formula (I).

Compounds of the formula (XXXIV) may be synthesised from compounds of the formula (XXXV):

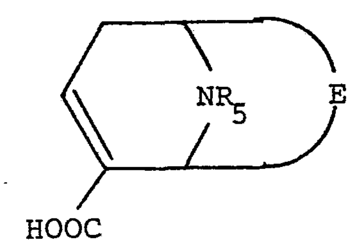

(XXXV)

wherein the variables are as hereinbefore defined, by the following conventional steps:

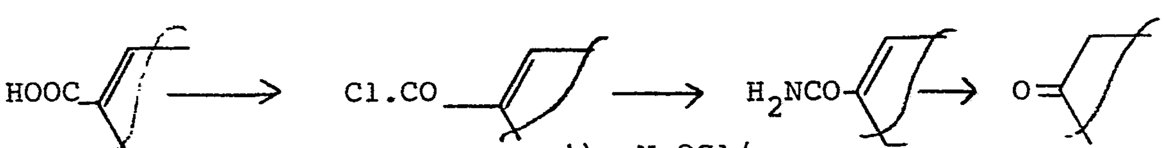

(XXXV)

i) NaOCl/
MeOH
ii) $H^+$

(XXXIV)

(remainder of rings omitted for clarity)

Compounds of the formula (XXXV) may be synthesised from compounds of the formula (XXXVI):

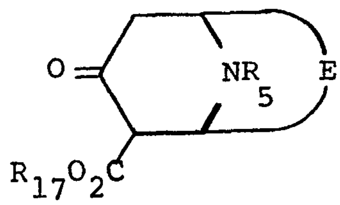

(XXXVI)

wherein $R_{17}$ is an organyl radical for example $C_{1-4}$ alkyl and the remaining variables are as hereinbefore defined, by the following conventional steps:

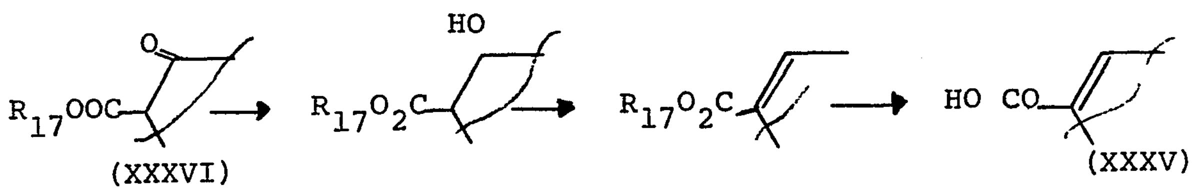

(XXXVI) (XXXV)

The dehydration step may be effected as described hereinbefore for compounds of formulae (XXIX).

Compounds of formula (XXXVI) wherein E is:

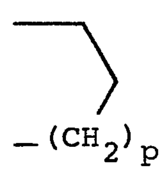

or

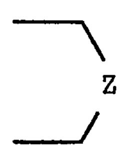

as defined may be prepared by the reaction of a compound of formula (XXXVII).

$$H_2N-R_5 \qquad \text{(XXXVII)}$$

wherein $R_3$ is as hereinbefore defined, with a corresponding dialdehyde of formula (XXXVIII):

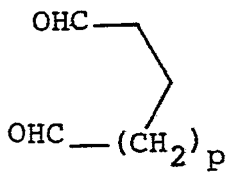

or

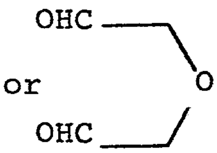

(XXXVIII)

and an acetone -1,3-dicarboxylic monoester, such as a $C_{1-4}$ alkyl monoester, and thereafter if necessary converting $R_5$ to $R_3$, by conventional methods.

The reaction is an example of the Robinson-Schopf reaction and is preferably carried out at a pH of 5 in a solution of sodium acetate buffer in water.

Compounds of formula (XXXVII) and 1,3-acetone dicarboxylic acid monoesters are known compounds.

The compounds of formula (XXXVIII) are known or can be prepared analogously to the preparation of known compounds.

Compounds of formula (XXXVI), wherein, when n = 0, one of $R_6$ and $R_7$ is $C_{1-4}$ alkyl and the other is hydrogen or $C_{1-4}$ alkyl, and wherein, when n = 1, $R_6$, $R_7$ and $R_8$ are as hereinbefore defined, and $R_5$ is as hereinbefore defined, can be prepared by reacting a known compound of formula

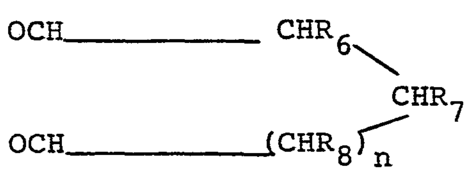

(XXXIX)

wherein $R_6$ to $R_8$ and n are as defined, with a compound of formula (XXXVII):

$$H_2N - R_5 \qquad \text{(XXXVII)}$$

wherein $R_5$ is as hereinbefore defined, and an acetone-1,3-dicarboxylic acid monoester under the same Robinson-Schopf conditions, and thereafter if desired converting $R_5$ to $R_3$.

Compounds of formula (XXXIX), wherein one of $R_6$ and $R_7$ is $C_{1-4}$ alkyl and the other is hydrogen or $C_{1-4}$ alkyl and n is 0, can be prepared by the hydrolysis of a compound of formula (XL):

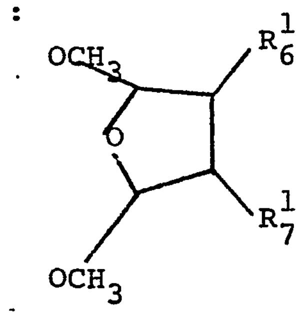

(XL)

wherein one of $R_6^1$ and $R_7^1$ is $C_{1-4}$ alkyl and the other is hydrogen or $C_{1-4}$ alkyl.

The compound of formula (XL) can be prepared by the catalytic hydrogenation of a compound of formula (XLI):

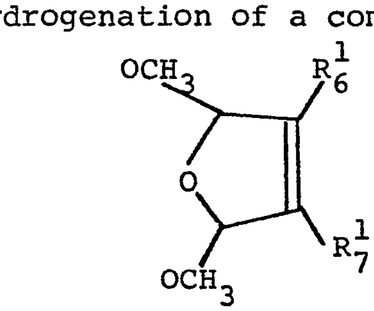

(XLI)

wherein $R_6^1$ and $R_7^1$ are as defined for formula (XL).

The catalytic hydrogenation is usually carried out with hydrogen and palladium on charcoal in methanol.

Compounds of formula (XXXVI), wherein one of $R_6$ and $R_7$ is hydroxy and the other is hydrogen or $C_{1-4}$ alkyl and n is 0, can be prepared by the acid hydrolysis of a compound of formula (XLII):

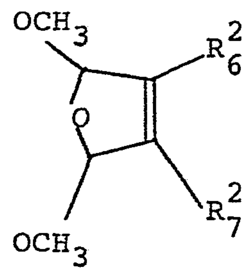

(XLII)

wherein one of $R_6^2$ and $R_7^2$ is hydrogen and the other is hydrogen or $C_{1-4}$ alkyl.

Compounds of formula (XLI) and (XLII) can be prepared from a compound of formula (XLIII):

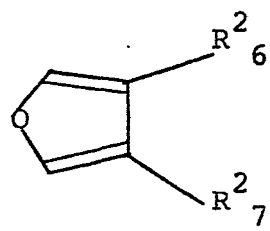

(XLIII)

wherein $R_6^2$ and $R_7^2$ are either respectively $R_6^1$ and $R_7^1$ as defined for formula (XL) or are respectively $R_6^2$ and $R_7^2$ as defined for formula (XLII), by reaction with bromine and methanol followed by basification.

The reaction of a compound of formula (XLIII) with bromine and methanol is normally carried out at about -40$^{o}$C, and the subsequent basification is carried out with ammonia or sodium carbonate.

Compounds of formula (XXXIX), wherein n is 1 and $R_6$, $R_7$ and $R_8$ are as hereinbefore defined, can be prepared by acidolysis of a compound of formula (XLIV):

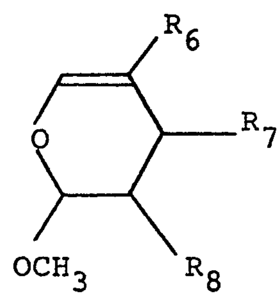

(XLIV)

wherein $R_6$ to $R_8$ are as hereinbefore defined, when n = 1, in accordance with the procedure described by K. Alder et al in Annal., 626, 73 (1959).

Compounds of formula (XLIV) are known compounds or are derivable therefrom or can be prepared analogously to the preparation of known, structurally-similar compounds.

The compounds of the formula (I) are dopamine agonists. Some are also α-adrenoceptor agonists.

The compounds may act at both presynaptic and postsynaptic receptors. Depending on their balance between action at presynaptic dopamine autoreceptors and action at postsynaptic dopamine receptors respectively, the compounds may be used in the treatment of central nervous system disorders related to excess dopamine release, such as psychosis, for example schizophrenia, and Huntington's chorea, and hyperkinetic and dyskinetic disorders, or in the treatment of central nervous system disorders related to deficient dopamine release, such as Parkinson's disease and hyperprolactinaemic syndromes.

As dopamine agonists, the compouns may also be used as mood modifiers in the treatment of CNS disorders, in particular the treatment of anxiety.

In common with other agonists, including the (-) enantiomer of the presynaptically acting dopamine agonist 3-PPP, compounds of the formula (I) may be dopamine antagonists, in particular acting post-synaptically, at higher doses, that is at doses towards the upper end of the dosage ranges mentioned hereinafter. As post-synaptic dopamine antagonists the compounds may be used in the treatment of dopamine-excess related disorders, such as described above.

The particular profile of any given compound may be readily ascertained by routine pharmacological tests of the types described hereinafter or well-known to the skilled man. Thus for example agonist activity at presynaptic autoreceptors may be ascertained by the compound's inhibition of spontaneous climbing behaviour in the mouse; agonist activity at postsynaptic receptors by an increase in spontaneous climbing behaviour in the mouse; and antagonist activity at postsynaptic receptors by inhibition of apomorphine (dopamine agonist) induced climbing in the mouse.

Compounds of the formula (X) are believed to have an activity profile in which presynaptic agonist activity predominates, in some cases to the extent that they may be regarded as selectively presynaptic.

The invention therefore also provides a pharmaceutical composition comprising a compound of the formula (I), a pharmaceutically acceptable salt thereof, or a solvate of the compound or its salt, together with a pharmaceutically acceptable carrier. Such compositions may be adapted for oral or parenteral administration, and as such may be in the form of tablets, capsules, oral liquid preparations, powders, granules, lozenges, reconstitutable powders, injectable and infusable solutions or suspensions; the compositions may also be in the form of suppositories. Normally, orally adminsitrable compositions are preferred.

Tablets and capsules for oral administration may be in unit dose presentation form, and may contain conventional excipients such as binding agents, fillers, tabletting lubricants, disintegrants and acceptable wetting agents and the like. The tablets may be coated according to methods well known in normal pharmaceutical practice. Oral liquid preparations may be in the form of, for example, aqueous or oily suspension, solutions, emulsions, syrups or elixirs, or may be presented in a dry product of reconstitution with water or other suitable vehicle before use. Such liquid preparations may contain conventional additivies such as suspending agents, emulsifying agents, non-aqueous vehicles (which may include edible oils), preservatives, and if desired conventional flavouring or colouring agents.

For parenteral administration, fluid unit dosage forms are prepared utilising the compound of the formula (I) and a sterile vehicle. The compound, depending on the vehicle and concentration used, can be either suspended or dissolved in the vehicle. In preparation solutions the compound can be dissolved for injection and filter sterilisied before filling into a suitable vial or ampoule and sealing. Advantageously, adjuvants such as a local anaesthetic, preservatives and buffering agents can be dissolved in the vehicle. Parenteral suspensions are prepared in substantially the same manner except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the compound.

As is common practice, the compositions will usually be accompanied by written or printed directions for use in the medical treatment concerned.

It will of course be realised that the precise dosage used in the treatment of any of the hereinbefore described disorders will depend on the actual compound of the formula (I) used, and also on other factors such as the seriousness of the disorder being treated.

The invention further provides a method of treatment of dopamine-dependent CNS disorders in mammals including humans comprising the administration of an effective amount of a compound of the formula (I) or a pharmaceutically acceptable salt thereof, or a solvate of compound or salt. The "effective amount" will depend in the usual way on a number of factors such as the nature and severity of the malady to be treated, the weight of the sufferer, and actual compound used.

However by way of illustration, unit doses will suitably contain 0.01 to 20mg of the compound of formula (I), for example 0.02 to 10mg.

Again by way of illustration, such unit doses will suitably be administered more than once a day, for example, 2, 3, 4, 5 or 6 times a day, in such a way that the total daily dose is suitably in the range 0.0005 to 10mg/kg per day.

The following Examples illustrate the preparation of compounds of formula (I), and the following Descriptions illustrate the preparation of intermediates therefor.

The exo- or endo-stereochemistry of the following compounds has been ascribed on the basis of analogous $^{1}H$ nmr data or preparative processes. In a case of unintentional nomenclature error, the $^{1}H$ nmr data and/or the preparative process is to be taken as definitive of structure.

Description 1

8-Benzyl-8-azabicyclo(3.2.1)oct-2-ene-2-carboxylic acid hydrochloride (D1)

A solution of methyl 8-benzyl-8-azabicyclo (3.2.1.) oct-2-ene-2-carboxylate (19.0g) in 5N-hydrochloric acid (250 ml) was heated under reflux for 20h under nitrogen and evaporated to drynesss *in vacuo* to give the carboxylic acid (20g) as a foam.

The NMR spectrum($(CD_3)_2SO$) showed the absence of a singlet at *ca* $\delta$ 4 corresponding to the starting material methyl ester.

Description 2

8-Benzyl-8-azabicyclo(3.2.1)oct-2-ene-2-carboxamide (D2)

A suspension of 8-benzyl-8-azabicyclo (3.2.1.) oct-2-ene-2-carboxylic acid hydrochloride (18g) in chloroform (500ml) and DMF (0.2ml) was treated dropwise with oxalyl chloride (20g), and stirred at room temperature for 3h, and evaporated to dryness *in vacuo* to afford the acid chloride ($\nu_{max}$ 1740 $cm^{-1}$), as a yellow oil which was cooled to -70$^oC$ (acetone-dry ice) and treated with a (35%) solution of ammonia in water (400ml), and allowed to warm up to room temperature. After stirring at room temperature for 2h solid potassium carbonate (ca. 100g) was added and the mixture was extracted with dichloromethane (6 x 70ml). The organic layer was washed with water (10ml), dried ($Na_2SO_4$), and evaporated to dryness *in vacuo*, to afford the carboxamide (15g) which crystallised from $CH_2Cl_2$-$Et_2O$ m.p. 143-145$^oC$. $\nu_{max}$ (Nujol) 3380, 3200 and 1660 $cm^{-1}$.

(Found: C, 73.15; H, 7.4; N, 11.8. $C_{15}H_{18}N_{2}O \cdot 0.25H_2O$ requires C, 73.0; H, 7.55; N, 11.35%).

Description 3

8-Benzyl-8-azabicyclo(3.2.1)octan-2-one (D3)

A solution of carboxamide (D2 8.0g) in methanol (400ml) at $-10^{o}C$ was treated dropwise with a 0.8N solution of sodium hypochlorite (45ml) over 15 min. maintaining the temperature below $-50^{o}C$. The solution was allowed to warm up to $+5^{o}C$ (over 0.5h) and was then heated at $70^{o}C$ for 15 min. Concentrated hydrochloric acid (20ml) was added and the solution was heated under reflux for 0.5h and evaporated to dryness *in vacuo*. The residue was treated with water (40ml) and solid potassium carbonate and the mixture was extracted with methylene chloride (5 x 50ml), washed with water (10ml) dried ($Na,SO_4$), evaporated to dryness *in vacuo* and the product was chromatographed on silica gel (100g) in dichloromethane to afford the 2-ketone (5.5g) m.p. $39-41^{o}C$ (from ether-hexane).

$\nu_{max}$ 1718 $cm^{-1}$; $\delta$ ($CDCl_3$) 1.5 - 2.5 (8H, m), 3.35 (2H, br.s), 3.7 (2H, s) and 7.3 (5H, br.s).

(Found: C, 76.3; H, 7.95; N, 6.35. $C_{14}H_{17}NO$ $0.33H_2O$ requires C, 76.0; H, 8.05; N, 6.33%).

Description 4

8-benzyl-2-(3'-methoxyphenyl)-8-azabicyclo(3.2.1)oct-2-ene (D4)

A solution of *m*-bromanisole (0.1 mol) in dry THF (10ml) is added slowly to a mixture of dry magnesium (0.125 mol) and 1 crystal of iodine in dry THF (25ml) at room temperature and the mixture is stirred for 2 hours, when most of the magnesium has been consumed. The solution is cooled to $-10^{o}C$ and a solution of the ketone (D3) (0.05 mol) in dry THF (10ml) is added dropwise. The mixture is allowed to stir at room temperature for 0.5 hour and then heated under reflux overnight. The product is acidified with an excess of 5N-hydrochloric acid and evaporated to dryness *in vacuo*. The residue is basified (sodium carbonate)

extracted with dichloromethane, dried, and evaporated to dryness to afford crude olefin (D4).

Description 5

(±)-(2-Exo) hydroxy and (2-endo) hydroxy-8-benzyl-2-(3'-methoxy-phenyl)-8-azabicyclo(3.2.1)octanes (D5) and (D6)

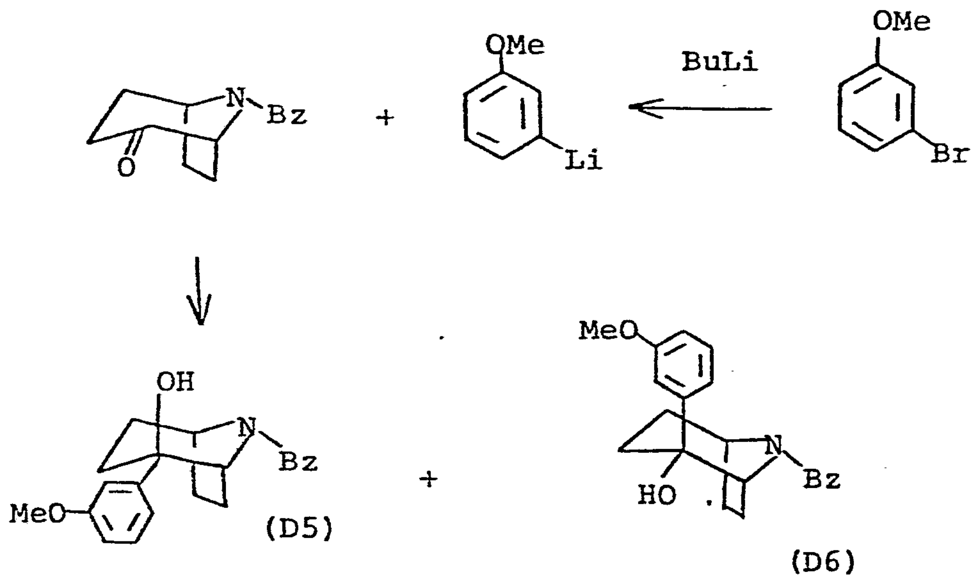


(D5) + (D6)

A solution of meta-bromoanisole (2.0g) in dry THF (25ml) under nitrogen at -55°C was treated with a solution of n-butyl lithium (1.15M; 14ml) in hexane and the solution was allowed to stir at this temperature for 2h. A solution of 8-benzyl-8-aza-bicyclo(3.2.1)-octan-2-one (1.0g) in dry THF (5ml) was added dropwise and the solution was allowed to warm up to room temperature (over 2h). The solution was quenched by the cautious addition of water, and evaporated to dryness *in vacuo*. The residue was dissolved in 5N-hydrochloric acid (50ml) and extracted with ether (3 x 40ml). The aqueous solution was basified (10% sodium carbonate) and extracted with dichloromethane (3 x 50ml). The organic fraction was washed with water, dried ($Na_2SO_4$) and evaporated to dryness to afford a mixture of the

*exo* (D5) and *endo* (D6) alcohols as an oil (1.15g). $\nu_{max}$ (film) 3440 $cm^{-1}$; δ ($CDCl_3$) 1.15-2.4 (8H, m), 2.85 (m) and 3.15 (m) (total 2H), 3.45 (s) and 3.50 (s) (total 2H), 3.80 (3H, s), 4.65 (1H, brs) (exchanges with $D_2O$), and 6.7-7.45 (9H, m).

Crystallisation of the crude product from ether-pentane afforded pure 2-exo-alcohol (D5), m.p. 70-71°C, (Found: C, 77.9; H, 7.9; N, 4.35. $C_{21}H_{25}NO_2$ requires C, 78.0; H, 78.0; H, 7.8; N, 4.35%),γ max.($CHCl_3$ solution) 3375 $cm^{-1}$; *m/e* 323(4%), 306 (4), 232 (25), 173 (25), 173 (35), 160 (50) and 91(100).

2-exo- and 2-endo-8-n-propyl-2-hydroxy-2-(3'-methoxyphenyl)-8-azabicyclo[3.2.1]octanes (D7) and (D8), 2-exo- and 2-endo-8-iso-propyl-2-hydroxy-2-(3'-methoxyphenyl)-8-azabicyclo [3.2.1]octanes (D9) and (D10), 2-exo- and 2-endo-8-phenethyl-2-hydroxy-2-(3'-methoxyphenyl)-8-azabicyclo[3.2.1]octanes (D11) and (D12), and 2-exo- and 2-endo-8-(2-thienylmethyl)-2-hydroxy-2-(3'-methoxyphenyl)-8-azabicyclo[3.2.1]octanes (D13) and (D14) are prepared analogously.

Example 1

(±)-8-Benzyl-2-(3'-methoxyphenyl)-8-azabicyclo(3.2.1)octane (1)

A solution of the olefin (D4) (0.1 mole) in ethanol (100ml) and $PtO_2$ (0.1g) is hydrogenated at atmospheric pressure and room temperature until uptake of hydrogen ceases. The mixture is filtered (celite) and evaporated to dryness *in vacuo* to afford crude (1) which is purified by crystallisation.

Example 2

± (2-Exo) and (2-Endo)-8-Benzyl-2-(3'-methoxyphenyl)-8-azabicyclo(3.2.1)octanes (2) and (3)

(A)

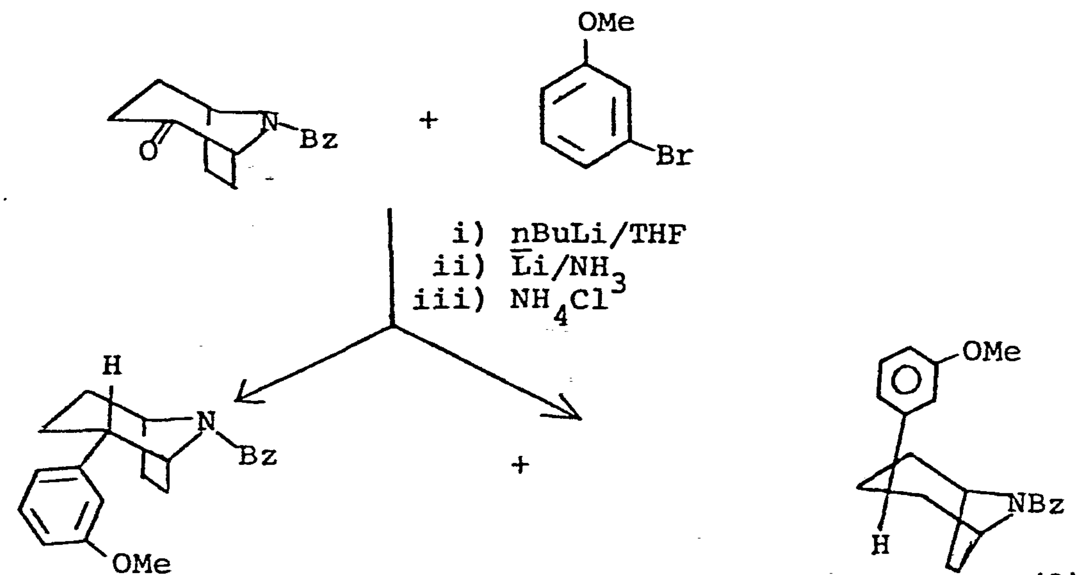


endo (3) exo (2)

To a stirred solution of m-bromoanisole (1.75g) in dry tetrahydrofuran (THF) (25ml) at -55°C under nitrogen was slowly added a 1.15 M solution of n-butyl lithium in hexane (8.15ml) over 0.15h. The solution was maintained at this temperature for 2h and then a solution of 8-benzyl-8-azabicyclo(3.2.1)octan-2-one (1.15g) in dry THF (10ml) was added dropwise and after the addition was complete the solution was allowed to reach room temperature (over 1.5h). The solution was cooled to -78°C and lithium foil (0.5g, ca 20 pieces) was added, followed by liquid ammonia (ca 50ml). After 10 min. a blue colour appeared and after 2 min. ammonium chloride (5g) was added cautiously to quench the reaction. The mixture was allowed to warm up to

room temperature and the solvents were allowed to evaporate to dryness. The residue was treated with 2N-sodium carbonate and extracted (5x) with ether. The combined ether extract was washed with water, dried ($Na_2SO_4$), and evaporated to dryness. The residue was chromatographed in ethyl acetate-pentane (1:19) on a model 7924 Harrigon Chromatotron using silica gel BF 254-gypsum absorbent (2mm), to afford the least polar exo-isomer (2) (0.15g) as an oil. (Found m/e 307.1938. $C_{21}H_{25}NO$ requires M 307.1936), νmax. (film) No bands above 3050cm$^{-1}$ or at 1718cm$^{-1}$; δ (250MHz-$CDCl_3$) 1.43(1H, m), 1.74 (3H, m), 1.9-2.3(4H, m), 2.71 (1H, br.d, J=6Hz), 3.25 (2H, m), 3.36 (1H, d, J=12Hz), 3.43(1H, d, J=12Hz), 3.75(3H,s), 6.75(1H,m) and 7.0-7.3(8H,m); m/e 307 (50%), 278(5), 216(10), 186(7), 712(25), 158(80) and 91(100).

Treatment with ethanolic-hydrogen chloride and recrystallisation from ethanol-ether gave the hydrochloride salt m.p. 150-151°C, (Found: C, 73.3; H, 7.8; N, 4.1, Cl, 10.4. $C_{21}H_{25}NO.HCl$ requires C, 73.35, H, 7.6; N, 4.05; Cl, 10.3%).

Further elution afforded the more polar endo-isomer (3) (0.33g) as an oil (Found m/e 307.1942. $C_{21}H_{25}NO$ requires M 307.1936), $\nu_{max.}$ (film). No bands above 3050 cm$^{-1}$ or at 1718 cm$^{-1}$; δ (250MHz, $CDCl_3$) 1.5-2.1 (8H,m), 3.1-3.32 (3H, m), 3.63 (2H, s), 3.79 (3H, s), 6.75 (3H, m) and 7.15-7.5 (6H,m); m/e identical to the exo-isomer.

Treatment with ethanolic-hydrogen chloride and recrystallisation from ethanol-ethyl acetate gave the hydrochloride salt hemihydrate, m.p. 166-168°, (Found: C, 71.3; H, 7.5; N, 3.95, Cl, 10.2. $C_{21}H_{25}NO.HCl\ 0.5H_2O$ requires C, 71.45; H, 7.7; N, 3.95; Cl, 10.05%).

(B)

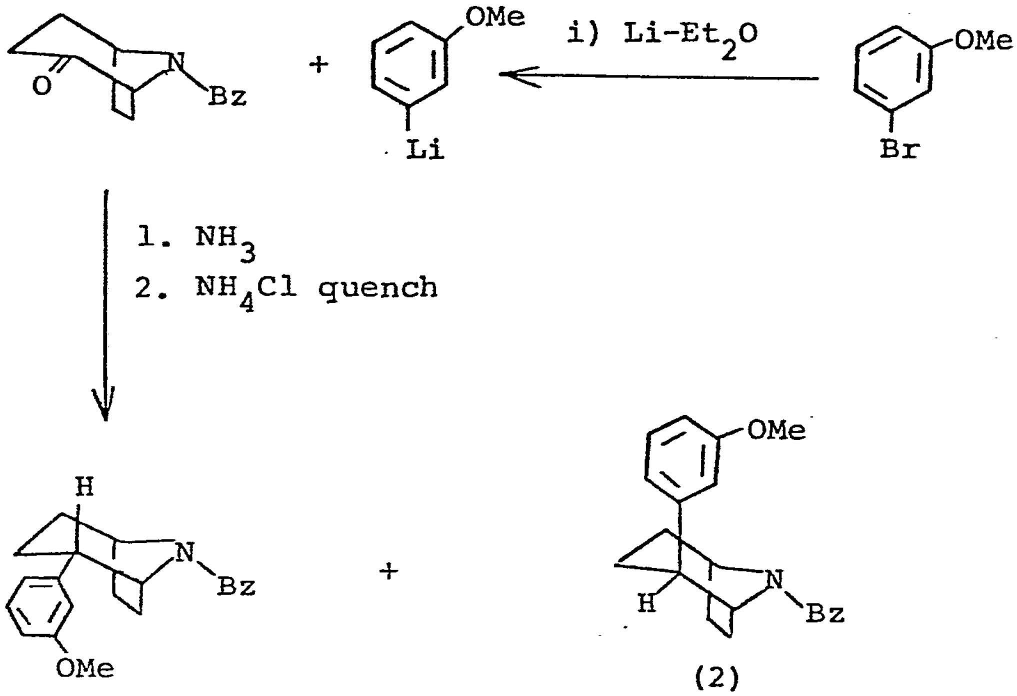


(3) (2)

Lithium (0.52g, <u>ca</u> 25 pieces) was added to a solution of <u>meta</u>-bromanisole (1.74g) in anhydrous ether (40ml) under nitrogen and after 3h at room temperature a solution of 8-benzyl-8-aza-bicyclo(3.2.1)octan-2-one (1.0g) in anhydrous ether (10ml) was added and the mixture was heated under gentle reflux for 4h, and then left at room temperature overnihgt. Liquid ammonia (<u>ca</u> 50ml) was then added and after formation of a blue colour, the reaction was quenched with ammonium chloride (6g). The product was recovered and chromatographed as described before, to afford the <u>exo</u> isomer (2) (0.02g) and endo isomer (3) (0.04g) identical n.m.r. and mass spectrum to those described above.

2-exo- and 2-endo-8-n-propyl-2-(3'-methoxyphenyl)-8-aza-bicyclo[3.2.1]octanes (4) and (5).

2-exo- and 2-endo-8-iso-propyl-2-(3'-methoxyphenyl)-8-aza-bicyclo[3.2.1]octanes (6) and (7), 2-exo- and 2-endo-8-phenethyl-2-(3'-methoxyphenyl)-8-aza-bicyclo[3.2.1]octanes (8) and (9), and 2-exo- and 2-endo-8-(2-thienylmethyl)-2-(3'-methoxyphenyl)-8-azabicyclo[3.2.1]octanes (10) and (11) are prepared analogously.

The above compounds (2) to (11) may also be prepared by analogous reductive steps on the alcohol intermediates (D5) to (D14).

Example 3

(±)-8-benzyl-2-(3'-hydroxyphenyl)-8-azabicyclo(3.2.1)octane hydrobromide (12)

A solution of (1) (0.5g) in 48% hydrobromic acid (50ml) is heated at 110$^{o}$C for 2hr under nitrogen, and evaporated to dryness *in vacuo*. The residue is crystallised from ethanol/ether to afford the phenol hydrobromide (12).

Example 4

(±)-(2-Exo)- and (2-endo)-8-Benzyl-2-(3'-hydroxyphenyl)-8-azabicyclo(3.2.1)octane hydrobromides (13) and (14)

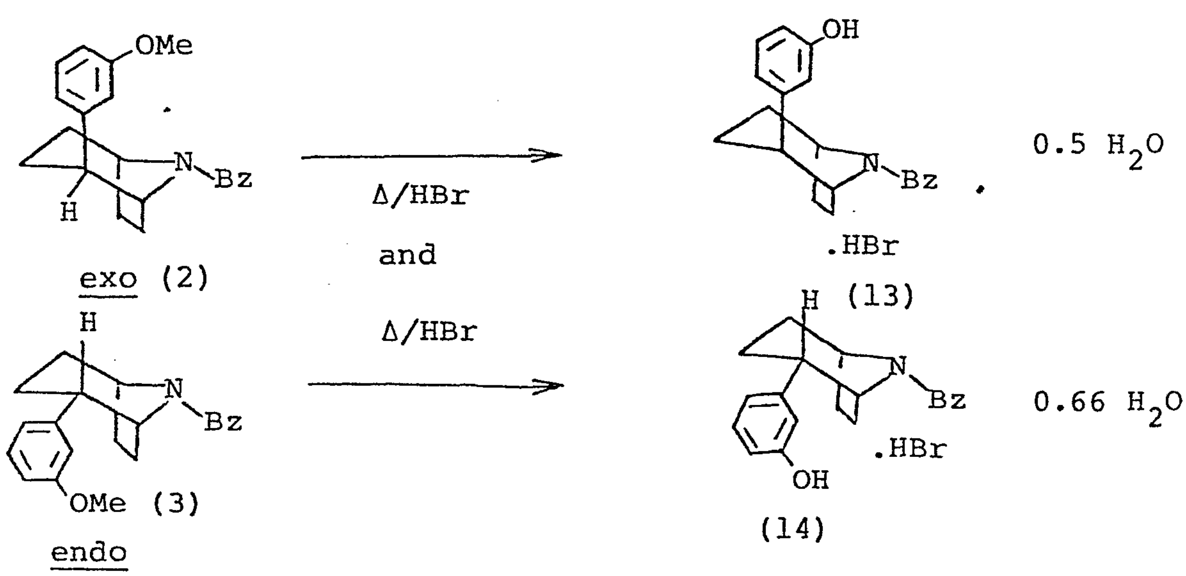


exo (2)

.HBr 0.5 $H_2O$

(13)

(3)

endo

.HBr 0.66 $H_2O$

(14)

a) A solution of exo-8-benzyl-2-(3'-methoxy phenyl)-8-azabicyclo(3.2.1)octane (2) (0.13g) in 48% aqueous hydrobromic acid (40ml) was heated under nitrogen at 110°C for 2h, and then evaporated to dryness in vacuo. The residue crystallised from ethanol-ether to afford the exo-phenol hydrobromide (13)(0.15g), m.p. 225-228°C, (Found: C, 62.65; H, 6.4; N, 3.65, Br, 21.35. $C_{20}H_{23}NOHBr$ 0.5$H_2O$ requires C, 62.65; H, 6.55; N, 3.65; Br, 20.85%; m/e 293.1773. $C_{20}H_{23}NO$ requires M 293.1780).

δ [$CDCl_3$ + $(CD_3)_2SO$] 1.65-2.85 (9H, m), 3.55-4.2 (3H, m), 4.45 (s) and 4.65 (s) (total 1H), 6.35 (1H, d, J=12Hz), 6.6-7.45 (8H, m), 7.7 (1H, br.s) and 9.0 (1H, s).

Isomer (13) showed $R_f$ 0.64 on a silica gel (Eastman) t.l.c. plate run in ethyl acetate.

b) A solution of endo-8-benzyl-2-(3'-methoxy phenyl)-8-azabicyclo(3.2.1)octane (3) (0.3g) in 48% aqueous hydrobromic acid (40ml) was heated under nitrogen at 110°C for 2h, and evaporated to dryness in vacuo. The residue crystallised from ethanol-ether to afford the endo-phenol hydrobromide (14) (0.28g), m.p. 133-137°C, (Found: C, 61.65; H, 6.2; N, 3.6. $C_{20}H_{23}NO$. HBr. 0.66$H_2O$ requires C, 62.2; H, 6.6; N, 3.65%; m/e 293.1775. $C_{20}H_{23}NO$ requires M 293.1780).

δ [$CDCl_3$ + $(CD_3)_2SO$] 1.6-2.5 (8H, m), 3.7-4.5 (5H, m), 6.7 (3H, m), 7.15 (1H, m), 7.5 (3H, m), 7.85 (2H, m), 9.1 (1H, s) and 10.4 (1H, br.s).

Isomer (14) showed $R_f$ 0.59 on silica gel (Eastman) t.l.c. plate run in ethyl acetate.

2-exo-8-n-propyl-2-(3'-hydroxyphenyl)-8-aza-bicyclo[3.2.1]octane (15),2-exo- and 2-endo-8-iso-propyl-2-(3'-hydroxyphenyl)-8-aza-bicyclo[3.2.1]octanes (17) and (18), 2-exo-8-phenethyl-2-(3'-hydroxyphenyl)-8-azabicyclo[3.2.1] octane (19), 2-exo

and 2-endo-8-(2-thienylmethyl)-2-(3'-hydroxyphenyl)-8-azabicyclo[3.2.1]octanes (21) and (22) are prepared analogously, as their hydrobromides.

The hydrobromide hydrate (16) of 2-endo-8-n-propyl-2-(3'-hydroxyphenyl)-8-azabicyclo[3.2.1]octane (16) was similarly prepared from (5a) - see Example 6 - and had the following characteristics, m.p. 210-215$^{o}$C (Found: C, 55.6; H, 7.05; N, 4.35, m/e 245.1786. $C_{16}H_{23}NO$. HBr.$H_2O$ requires: C, 55.8: Hm 7.6; N, 4.05'. $C_{16}H_{23}NO$ requires M 245.1780).

The hydrobromide 1.5 hydrate (20) of 2-endo-8-phenethyl-2-(3'-hydroxyphenyl)-8-azabicyclo[3.2.1]octane (20) was similarly prepared from (9a) - see Example 8 - and had the following characteristics m.p 102-106$^{o}$C (Found: C, 60.3; H, 6.6; N, 3.8, m/e 307.1919. $C_{21}H_{25}NO$, HBr, 1.5 $H_cO$ requires: C, 60.7; H, 7.05; N, 3.35%. $C_{21}H_{25}NO$ requires M 307.1889).

Example 5

±-(2-exo) and (2-endo)-2-(3'-methoxy-phenyl)-8-azabicyclo-(3.2.1)-octane hydrochlorides (23) and (24)

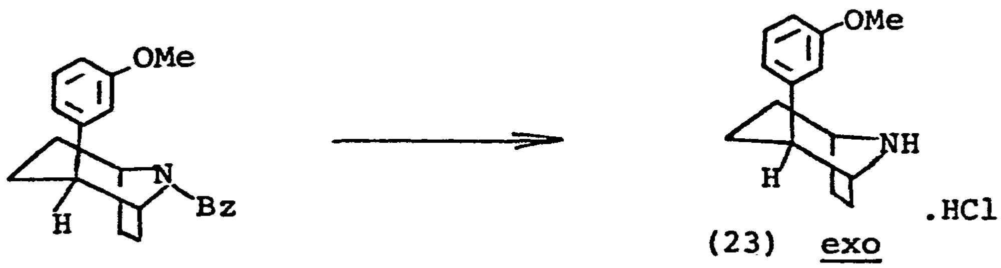

(2) exo    (23) exo

and

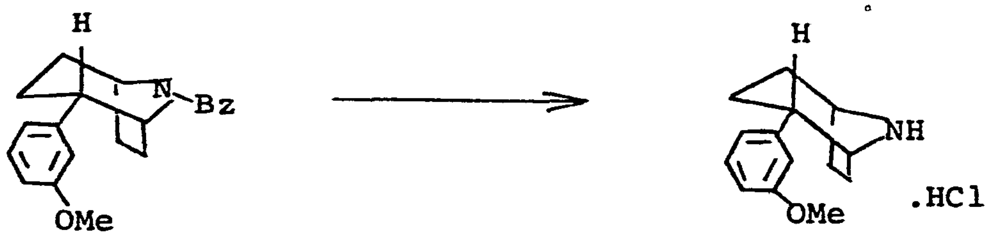

(3) endo    (24) endo

(a) A solution of (2-exo)-8-benzyl-2-(3'-methoxyphenyl)-8-azabicyclo(3.2.1)octane (2) (0.65g) in ethanol (50ml) was hydrogenated over 10% palladium-charcoal (0.5g) at room temperature for 4 days. The solution was filtered (celite), evaporated to dryness *in vacuo*, treated with ethanolic-hydrogenchloride and crystallised from ethanol-ether to afford (2-exo)-2-(3'-methoxyphenyl)-8-azabicyclo(3.2.1) octane hydrochloride (23), (0.15g) m.p. 136-140°C, (Found: *m/e* 217.1473. $C_{14}H_{19}NO$ requires *M* 217.1466). δ(CDCl3) 1.5-2.7 (8H, m), 3.0 (1H, br.s), 3.85 (3H, s), 4.05 (2H, br.s), and 6.75-7.4 (4H, m).

(b) The 2-endo isomer (3) (0.65g) was similarly converted but using a hydrogenation time of 18 hours to 2-(endo)-2-(3'-methoxyphenyl)-8-acabicyclo(3.2.1)-octane hydrochloride (24) (0.47g). m.p. 165-166°C, (Found: C, 65.95; H, 7.95; N, 5.5; Cl, 13.95. *m/e* 217.1465 $C_{14}H_{19}NO$ HCl requires C, 66.25; H, 7.95; N, 5.5; Cl, 13.95%. $C_{14}H_{19}NO$ requires *M* 217.1466); δ ($CDCl_3$) 1.5-2.6 (8H, m), 3.65 (1H, m), 3.8 (3H, s), 4.2 (2H, br.s), 6.65-7.4 (4H, m) and 9.85 (2H, br.s).

0076089

<u>Example 6</u>

± <u>(2-Endo)-8-n-propyl-2-(3'-methoxyphenyl)-8-azabicyclo [3.2.1]octane hydrochloride</u> (5a)

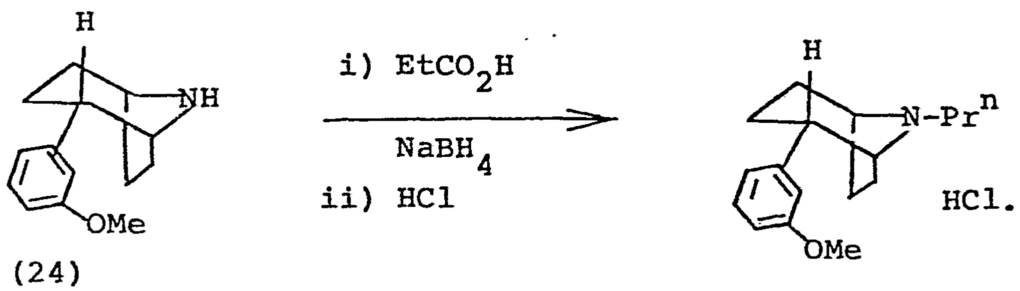


(24)

(5a) HCl salt of (5)

A solution of (2-endo)-2-(3'-methoxyphenyl)-8-azabicyclo [3.2.1]octane (0.25g) in propionic acid (5ml) was added to a mixture of propionic acid (10ml), dry toluene (10ml) and sodium borohydride (0.5g) and the mixture was heated under reflux for 3 hours. It was evaporated to dryness <u>in vacuo</u>, basified with sodium carbonate, extracted (3x) with dichloromethane. The organic fraction was washed with water, dried ($Na_2SO_4$), and evaporated to dryness <u>in vacuo</u>. The residue was treated with ethanolic-hydrogen chloride and recrystallised from ethyl acetate-ether to afford <u>(2-endo)-8-n-propyl-2-(3'-methoxyphenyl)-8-azabicyclo [3.2.1]octane hydrochloride</u> (5a)(0.245g), m.p. 180-182$^{\circ}$C, (Found: C, 68.5; H, 8.9; N, 4.75. $C_{17}H_{25}NO$ HCl requires C, 69.0; H, 8.85; N, 4.75%).

Example 7

(±)-(2-Endo)-8-Phenethyl-2-(3'-methoxyphenyl)-8-azabicyclo [3.2.1]octane hydrochloride (9a)

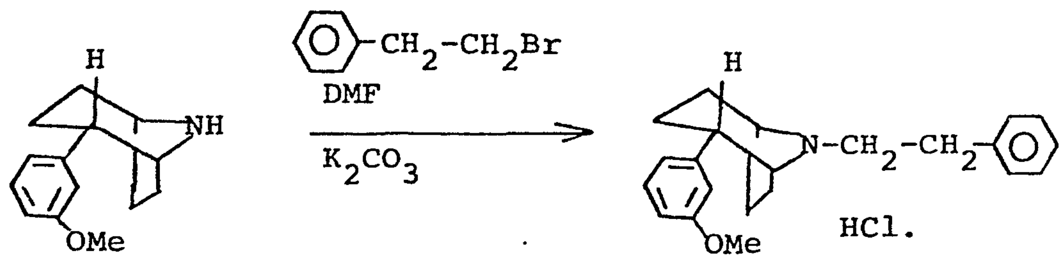


(24)

(9a) HCl salt of (9)

A mixture of (2-endo)-2-(3'-methoxyphenyl)-8-azabicyclo [3.2.1]octane (24) (0.4g), dry DMF (10ml), phenethyl-2-bromide (0.38g) and anhydrous potassium carbonate (2g) was heated at ca 70°C for 2 hour under nitrogen. The mixture was evaporated to dryness in vacuo, dissolved in 5N-hydrochloric acid, washed with ether, basified with 10% sodium carbonate and extracted (3x) with dichloromethane. The dichloromethane layer was washed with $H_2O$, dried ($Na_2SO_4$) evaporated to dryness in vacuo, treated with ethanolic-hydrogen chloride and crystallised from ethanol-ethyl acetate-ether to afford (2-endo)-8-phenethyl-2-(3'-methoxyphenyl)-8-azabicyclo[3.2.1]octane hydrochloride (9a) (0.55g), m.p. 145-146°C, (Found: C, 73.65; H, 7.84; N, 3.9; Cl, 10.05; m/e 321.2085. $C_{25}H_{27}NO$ HCl requires C, 73.85; H, 7.9; N, 3.9; Cl, 9.9%; $C_{22}H_{27}NO$ requires M 321.2093).

Biological Data

Tested compounds inhibit the spontaneous climbing behaviour of mice. It is believed that this inhibitory effect is mediated by stimulation of presynaptic dopaminergic autoreceptors, i.e. receptors whose stimulation results in inhibition of dopamine synthesis and electrical activity of dopamine neurones. Spontaneous climbing behaviour was assessed by the method of M.P. Martres et al., Brain Research, 1977, 136, 319. This involves the monitoring of the frequency of climbing behaviour of mice in inverted food hoppers over a 10 minute period commencing 3 minutes after subcutaneous administration of compound.

A climb is defined as the mouse having all four feet off the ground. Experiments are carried out with a minimum of 5 groups of 10 mice, one group acting as control. The number of climbs exhibited by the drug treated group is expressed as a percentage change from the number exhibited by the control group.

| Compound | % Inhibition of Spontaneous Climbing at 0.2mg/kg |
|---|---|
| 13 | 24 |
| 14 | 38 |
| 16 | 49 |
| 20 | 12 |

No toxic effects were observed in the above tests.

CLAIMS:

1. A compound of formula (I) or a pharmaceutically acceptable salt thereof or a solvate of the compound or its salt:

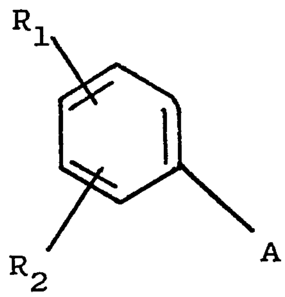

(I)

wherein:

$R_1$ is hydroxy, $C_{1-4}$ alkoxy or an in-vivo hydrolysable acyloxy group:

$R_2$ is hydrogen, $C_{1-4}$ alkyl, hydroxy, $C_{1-4}$ alkoxy or an in-vivo hydrolysable acyloxy group; or $R_1$ and $R_2$ together are $C_{1-2}$ alkylenedioxy;

A is a group

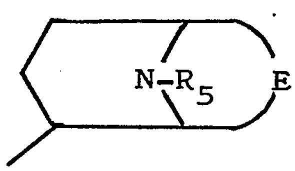

where E is

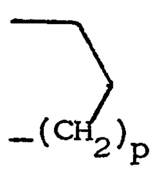

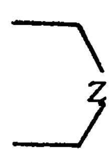

or

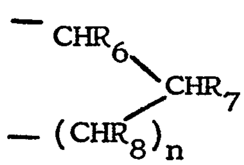

where

p is 0 to 2;
Z is O or S;
n is 0 or 1; and
one of $R_6$ and $R_7$ when n=0 is $C_{1-4}$ alkyl and the other is hydrogen or $C_{1-4}$ alkyl or one of $R_6$, $R_7$ and $R_8$ when n=1 is $C_{1-4}$ alkyl and the other two are the same or different and are hydrogen or $C_{1-4}$ alkyl; and

$R_5$ is $C_{1-7}$ alkyl; a group $-(CH_2)_sR_{13}$ where s is 0 to 2 and $R_{13}$ is a $C_{3-8}$ cycloalkyl group; a group $-(CH_2)_tR_{14}$ where t is 1 or 2 and $R_{14}$ is $C_{2-5}$ alkenyl or a phenyl group optionally substituted by one or two substituents selected from $C_{1-4}$ alkoxy, trifluoromethyl, halogen, nitro, carboxy, esterified carboxy and $C_{1-4}$ alkyl optionally substituted by hydroxy, $C_{1-4}$ alkoxy, carboxy, esterified carboxy or in vivo hydrolysable acyloxy, or a thienyl group.

2. A compound according to claim 1 wherein E is

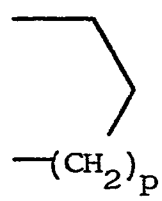

3. A compound according to claim 2 of formula (IX):

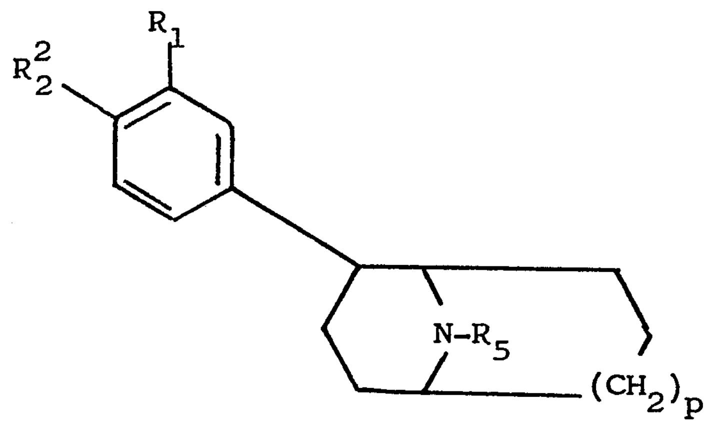

(IX)

wherein:

$R_2^2$ is hydrogen or $C_{1-4}$ alkyl and the remaining variables are as defined in claim 1.

4. A compound according to claim 3 of formula (X):

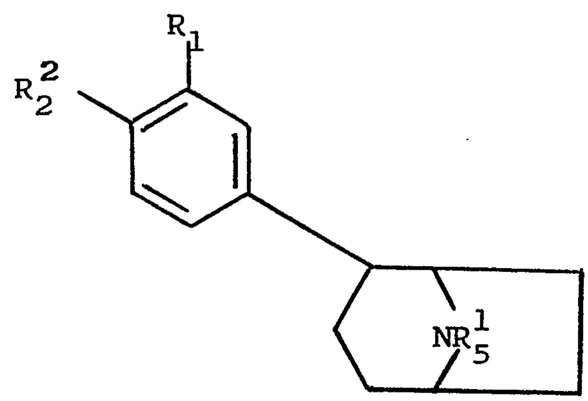

(X)

wherein $R_5^1$ is $C_{1-3}$ alkyl or allyl and $R_1$ and $R_2^2$ are as defined in claim 3.

5. A compound according to claim 3 of the formula (XII):

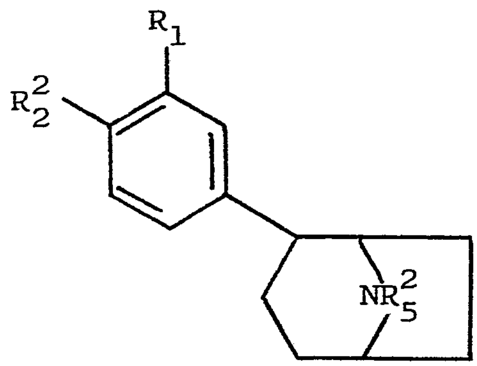

(XII)

wherein $R_5^2$ is a group $-(CH_2)_tR_{14}^1$ wherein t is 1 or 2 and $R_{14}^1$ is optionally substituted phenyl as defined in formula (I); or thienylmethyl; and $R_1$ and $R_2^2$ are as defined in claim 3.

6. (±)-2-exo-8-benzyl-2-(3′-hydroxyphenyl)-8-azabicyclo[3.2.1]octane hydrobromide,
(±)-2-endo-8-benzyl-2-(3′-hydroxyphenyl)-8-azabicyclo[3.2.1]octane hydrobromide,
(±)-2-endo-8-n-propyl-2-(3′-hydroxyphenyl)-8-azabicyclo[3.2.1]octane hydrobromide hydrate, or
(±)-2-endo-8-phenethyl-2-(3′-hydroxyphenyl)-8-azabicyclo[3.2.1]octane hydrobromide sesquihydrate

7. A process for the preparation of a compound according to claim 1 which process comprises the reduction of a compound of the formula (XXVIII):

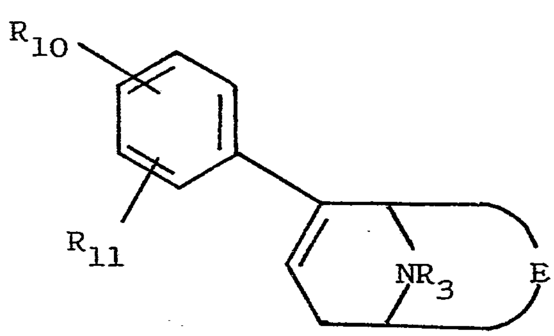

(XXVIII)

$R_{10}$ is hydroxyl or protected hydroxyl;

$R_{11}$ is hydrogen, $C_{1-4}$ alkyl, hydroxyl or protected hydroxyl;

$R_3$ is $R_5$ as defined in formula (I) or $C_{1-7}$ alkanoyl; a group $-CO(CH_2)_xR_{13}$ where x is 0 or 1 and $R_{13}$ is as defined in claim 1; or a group $-CO(CH_2)_yR_{14}$ where y is 0 or 1 and $R_{14}$ is as defined in claim 1;

and the remaining variable are as defined in claim 1,

and thereafter as necessary converting $R_3$ to $R_5$, as desired or necessary converting $R_{10}$ or $R_{11}$ to $R_1$ or $R_2$ respectively or $R_1$, $R_2$ or $R_5$ to other $R_1$, $R_2$ or $R_5$ and optionally salifying the resulting compound of the formula (I)

8. A process according to claim 7 wherein the compound of formula (XXVIII) is prepared in situ by the dehydration of a compound of formula (XXVIII)

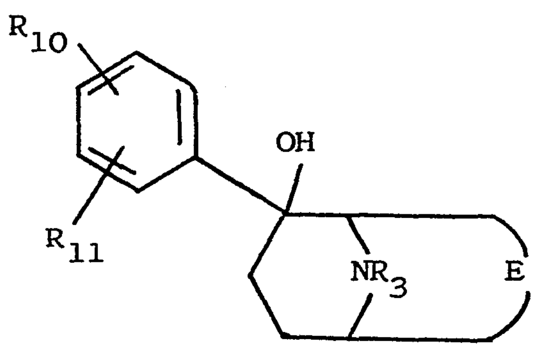

(XXVIII)

wherein the variables are as defined in claim 7.

9. A process for the preparation of a compound of formula (XXXI):

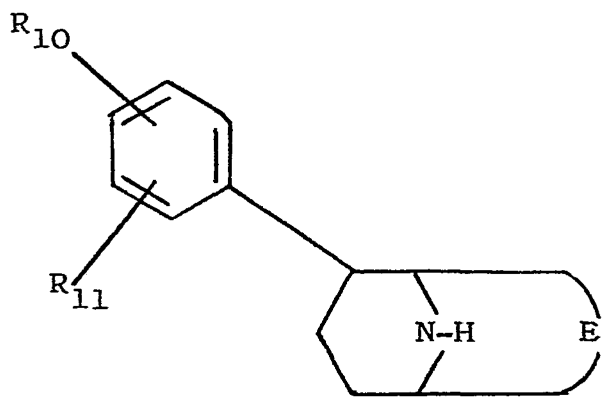

(XXXI)

with a compound of the formula (XXXII):

$$Q - R_3 \qquad \text{(XXXII)}$$

wherein:

$R_{10}$ is hydroxy or protected hydroxy;

$R_{11}$ is hydrogen, $C_{1-4}$ alkyl, hydroxy or protected hydroxy;

Q is a group readily displaceable by a nucleophile; and the remaining variables are as hereinbefore defined;

and as necessary converting $R_3$ to $R_5$;

as desired or necessary converting $R_{10}$ or $R_{11}$ to $R_1$ or $R_2$ respectively; converting $R_1$, $R_2$ or $R_5$ to other $R_1$, $R_2$ or $R_5$; and optionally salifying the resulting compound of the formula (I).

10. A pharmaceutical composition comprising a compound of the formula (I), a pharmaceutically acceptable salt thereof, or a solvate of the compound or its salt, together with a pharmaceutically acceptable carrier.

11. A method of treatment of dopamine-dependent CNS disorders in mammals including humans comprising the administration of an effective amount of a compound of the formula (I) or a pharmaceutically acceptable salt thereof, or a solvate of the compound or its salt.

12. A compound of formula (XXXI):

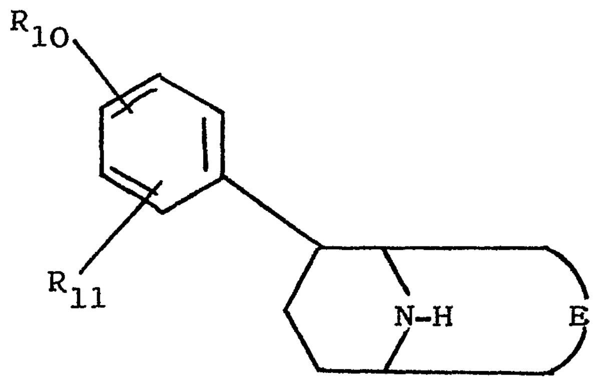

(XXXI)

wherein:

$R_{10}$ is hydroxy or protected hydroxy;

$R_{11}$ is hydrogen, $C_{1-4}$ alkyl, hydroxy or protected hydroxy;

and the remaining variables are as defined in claim 1.